# FASCICULE DE BREVET EUROPEEN

(11) **EP 1 076 720 B1**
(45) Date de publication et mention de la délivrance du brevet: **17.11.2010**
(21) Numéro de dépôt: 99915852.0
(22) Date de dépôt: 27.04.1999
(51) Int. Cl.: C12Q 1/68, C12N 9/08, C07K 14/245

(54) **SEQUENCES NUCLEOTIDIQUES POUR LA DETECTION DES ESCHERICHIA COLI ENTEROHEMORRAGIQUES (EHEC)**
NUKLEOTIDSEQUENZEN ZUM NACHWEIS VON ENTEROHÄMORRHAGISCHER ESCHERICHIA COLI
NUCLEOTIDE SEQUENCES FOR DETECTING ENTEROHEMORRHAGIC ESCHERICHIA COLI (EHEC)

(30) Priorité: 28.04.1998 FR 9805329
(43) Date de publication de la demande: 21.02.2001
(73) Titulaire: Bio-Rad Pasteur, 92430 Marnes-la-Coquette (FR)
(72) Inventeur: FRECHON, Dominique, Thérèse, Marie, F-75018 Paris (FR); LAURE, Françoise, Claudine, F-75020 Paris (FR); THIERRY, Dominique, F-92100 Boulogne (FR)
(74) Mandataire: Jacobson, Claude
(86) Numéro de dépôt international: PCT/FR1999/001000
(87) Numéro de publication internationale: WO 1999/055908

(56) Documents cités:
- WO-A-97/32043
- CA-A- 2 078 716
- US-A- 5 475 098
- US-A- 5 738 995
- MAKINO K ET AL.: "Complete nucleotide sequence of 93-kb and 3.3-kb plasmids of an enterohemorrhagic Escherichia coli O157:H7 derived from Sakai outbreak" DNA RESEARCH, vol. 5, no. 1, 28 février 1998 (1998-02-28), pages 1-9, XP002091199 TOKYO JP cité dans la demande
- BRUNDER W. ET AL.: "KatP, a novel catalase-peroxydase encoded by the large plasmid of enterohaemorrhagic Escherichia coli O157:H7" MICROBIOLOGY, vol. 142, no. 11, novembre 1996 (1996-11), pages 3305-3315, XP002091200 GB
- FRATAMICO P M ET AL: "DETECTION OF ESCHERICHIA COLI O157:H7 BY MULTIPLEX PCR" JOURNAL OF CLINICAL MICROBIOLOGY, vol. 33, no. 8, août 1995 (1995-08), pages 2188-2191, XP000197544 US
- BEEBAKHEE G ET AL: "CLONING AND NUCLEOTIDE SEQUENCE OF THE EAE GENE HOMOLOGUE FROM ENTEROHEMORRHAGIC ESCHERICHIA COLI SEROTYPE 0157:H7" FEMS MICROBIOLOGY LETTERS, vol. 91, 1 janvier 1992 (1992-01-01), pages 63-68, XP002040899 AMSTERDAM NL ISSN: 0378-1097

## Description

L'invention a pour objet deux séquences nucléiques d'origine plasmidique présentes chez les bactéries du groupe *Escherichia coli* entérohémorragiques (EHEC), l'utilisation desdites séquences pour la recherche des EHEC. notamment ceux possédant les gènes codant pour les facteurs de virulence entérohémolysine et intimine, et plus particulièrement la détection spécifique du sérotype O157 :H7. L'invention vise également un procédé mettant en oeuvre lesdites séquences ainsi que les trousses de détection les contenant.

Les bactéries du groupe EHEC appartiennent à la famille des *Escherichia coli* producteurs de vérotoxines ou VTEC, responsables de syndromes diarrhéiques dont les conséquences peuvent être fatales chez l'homme. En particulier, les EHEC peuvent engendrer des colites hémorragiques (CH), et éventuellement l'apparition de complications majeures comme le syndrome hémolytique et urémique (SHU) ou le purpura thrombotique thrombopénique *(*Griffin et Tauxe, Epidemiol. Rev. 13, 1991, 60-98*).*

Aussi, l'incidence de ces infections sur la santé publique est telle, qu'elle implique un contrôle accru des denrées alimentaires et des moyens de détections rapides, notamment en cas d'épidémies.

Plusieurs sérotypes, appartenant au groupe EHEC ont été identifiés et rendus responsables de différents foyers épidémiques: O157 :H7, O26 :H11. O111 :NM, 0103 :H2, 0145 :NM etc *(*Acheson et Keush, ASM News 62, 1996. 302-306). Cependant, c'est le sérotype 0157 :H7 qui a été le plus fréquemment isolé.

Les méthodes de détection traditionnelles consistent à identifier les bactéries ou à déceler les toxines sécrétées par celles-ci. La détection de *E. coli* O157 :H7 est principalement réalisée sur la base du sérotypage, associé à la recherche de propriétés métaboliques, comprenant l'absence de fermentation du sorbitol et/ou l'absence d'activité β-glucuronidase. Il n'existe pas, par ailleurs, de méthode bactériologique propre à la détection des EHEC, mais des tests permettant d'orienter le diagnostic. En particulier, l'utilisation de géloses supplémentées avec du sang ou des hématies lavées permet de mettre en évidence le caractère entérohémolytique, généralement présent chez les EHEC.

De manière générale, les méthodes bactériologiques et immunologiques relatives à la détection de *E. coli* O157 :H7 sont longues, fastidieuses relativement coûteuses et nécessitent une confirmation sérologique. Par ailleurs, ces méthodes ne permettent pas d'établir une identification de *E. coli* 0157 :H7 du fait des réactions croisées avec d'autres genres et espèces bactériens, ce qui rend l'interprétation difficile.

L'utilisation de sondes nucléiques est donc apparue comme une alternative à ces méthodes traditionnelles. Des efforts importants ont été réalisés pour développer des sondes, capables de détecter d'une manière sensible et spécifique les bactéries *E. coli* de type EHEC, impliquées dans les cas de CH et/ou SHU, et dont le prototype le plus répandu est *E. coli* O157 :H7.

En particulier, des sondes ou fragments, permettant la détection des gènes responsables de la virulence des *E. coli,* encore appelés facteurs de virulence, ont été publiés. Toutefois, aucun des facteurs de virulence actuellement connu ne permet à lui seul d'identifier des souches pathogènes de *E . coli* O157 :H7 ou des EHEC.

Ainsi, l'utilisation de sondes ou fragments nucléiques pour la détection des gènes codant pour les vérotoxines (*vt1* ou *st1, vt2* ou *st2*), décrite par de nombreux groupes de recherche *(*Karch et Meyer, J. Clin Microbiol. 27, 1989, 2751-2757*;* Gannon et al. , Appl. Env. Microbiol. 58, 1992, 3809-3815 *;* Begum et al., J. Clin. Microbiol. 31, 1993, 3153-3156 *;* Witham et al., Appl. Env. Microbiol. 62, 1996, 1347-1353*),* a montré que les gènes codant pour les vérotoxines sont associés aux souches bactériennes pathogènes *E. coli* 0157 :H7 et autres EHEC, mais peuvent aussi être présents chez des souches *E. coli* non pathogènes, ou éventuellement chez d'autres genres bactériens comme *Shigella dysenteriae, Citrobacter freundii,* etc.

De même, la protéine d'adhésion dénommée intimine est également impliquée dans la virulence des bactéries de type EHEC. Des sondes ont été notamment sélectionnées sur le gène correspondant (eae) par Gannon et al. dans J. Clin. Microbiol. 31, 1993, 1268-1274*,* Louie et al. dans Epidemiol. Infect. 112, 1994, 449-461 et Meng et al. dans Int. J. Food Microbiol. 32, 1996, 103-113*.* Cependant, bien que ce facteur de virulence soit étroitement associé au groupe des EHEC, il est également rencontré chez les *E*. *coli* entéropathogènes (EPEC) comprenant le sérotype 055 :H7.

Enfin, des sondes ont été sélectionnées sur un plasmide de 60 MDa, codant entre autres pour l'entérohémolysine, facteur de virulence également présent chez de nombreux EHEC *(*Levine et al., J. Infect. Dis. 156, 1987, 175-182 *;* Schmidt et al., Infect. Immun. 63, 1995, 1055-1061). Ainsi, le brevet US 5,475,098 vise les séquences nucléiques contenues dans l'opéron de l'entérohémolysine, correspondant aux gènes *hlyA, hlyB* et à la région intergénique *hlyA-hlyB.* Les séquences oligonucléotidiques revendiquées permettent une détection spécifique des EHEC, mais l'invention ne permet pas de différencier *E*. *coli* O157 :H7 des autres EHEC. Par ailleurs, le brevet US 5,652,102, décrit une séquence nucléique située sur un fragment de restriction issu du plasmide de 60 MDa. Cependant, l'utilisation d'oligonucléotides dérivés de cette séquence dans une réaction de polymérase en chaîne ou "Polymerase Chain Reaction" (PCR), ne permet pas à elle seule l'identification spécifique du sérotype 0157 :H7, et nécessite par conséquent l'utilisation conjointe d'amorces amplifiant les gènes codant pour les vérotoxines et l'intimine.

Le plasmise p0157, isolé d'une souche *E*. *coli* 0157:H7 provenant d'un échantillon clinique, a dernièrement été décrit dans sa totalité (Makino et al, DNA Research 5, 1998, 1-9). Une cartographie du plasmide représentant l'ordonnancement des différents gènes sur le génome du plasmide indique la présence de 186 phases ouvertes de lecture (ORF). Toutefois, l'absence de données de la séquence nucléique (données non disponibles au moment de la parution de l'article), ne permet en aucun cas d'identifier une région d'intérêt diagnostique pour la détection spécifique de *E. coli* 0157:H7.

Dernièrement, la demande de brevet WO 97/32045 vise des oligonucléotides sélectionnés à partir d'une séquence chromosomique obtenue par la méthode RAPD (Random Amplified Polymorphic DNA), conduisant à la détection d'environ 99,5 % de *E. coli* O157 :H7, mais les séquences nucléiques revendiquées détectent également près de 3 % de *E . coli* non EHEC, ce qui n'est pas satisfaisant en terme de spécificité, notamment dans le domaine de l'agro-alimentaire.

L'inconvénient majeur de tous ces systèmes de détection réside donc dans le fait qu'aucun d'entre eux, ne permet d'établir clairement et simplement l'identification du sérotype *E. coli* O157:H7. Il est en effet très souvent nécessaire d'associer plusieurs systèmes d'amplification et/ou de détection pour rendre le résultat précis. Les protocoles utilisés sont alors difficiles à mettre en oeuvre (amplifications multiples, simultanées) et les résultats obtenus quant à la sensibilité et à la spécificité sont fortement dépendants, non seulement des cibles nucléiques utilisées, mais aussi des conditions opératoires.

Or, comme il a été précédemment indiqué, ce sérotype peut provoquer de graves syndromes pouvant conduire à la mort, ce qui implique des moyens rapides et fiables de détection, notamment en cas d'épidémie.

Les travaux des inventeurs ont consisté à rechercher des séquences spécifiques à partir d'une banque génomique de *E. coli* O157 :H7, permettant la reconnaissance des principaux sérotypes de *E. coli* pathogènes pour l'homme, et plus particulièrement O157 :H7. La banque a été criblée contre *E. coli* entéropathogène 055 :H7, ancêtre supposé du sérotype O157 :H7, les deux génomes étant extrêmement proches d'après les analyses de polymorphisme réalisées par T. Whittam et al. dans Infect. Immun. 61, 1993, 1619-1629.

Ces travaux ont permis d'isoler deux fragments nucléiques d'intérêt pour la détection des EHEC et plus particulièrement pour la détection de *E. coli* O157 :H7, comprenant les séquences nucléiques SEQ ID N°1 et SEQ ID N°2. situées sur le plasmide entérohémolytique de 60 MDa. Les clones correspondants pDF3 et pDF4 contenant ces séquences ont été déposés auprès de la Collection Nationale de Cultures de Microorganismes de l'Institut Pasteur respectivement sous les numéros I-1999 et I-2000, le 26 mars 1998.

D'une façon surprenante, il a été mis en évidence une première séquence (SEQ ID N°1) comprenant l'association stable d'une partie de la séquence d'insertion IS*91* et de la séquence issue du gène *katP* de *E. coli* 0157 :H7 ou d'une partie de celle-ci, l'enchaînement nucléique en résultant, jamais décrit par ailleurs, étant spécifiquement retrouvé chez *E. coli* O157 :H7.

Le gène *katP,* codant pour une catalase-péroxydase, est présent sur le plasmide entérohémolytique de *E*. *coli* O157 :H7 et de nombreuses EHEC *(*Brunder et al., Microbiol. 142, 1996, 3305-3315*),* et la séquence d'insertion IS*91*, identifiée sur les plasmides α-hémolytiques de *E*. *coli (*Zabala et al., J. Bacteriol. 151, 1982, 472-476*),* n'a encore jamais été décrite chez les souches entérohémolytiques de type *E*. *coli* O157 :H7.

La mise en évidence d'une séquence d'insertion tronquée au niveau de la jonction IS*91-katP* (absence de la séquence inversée répétée gauche (IR_{L}) de l'IS*91*) suggère aussi une intégration stable de l'IS*91* dans cette partie du génome de *E. coli* O157 :H7.

L'analyse des produits amplifiés d'un grand nombre de souches 0157 :H7 d'origines diverses démontre la conservation de cet enchaînement nucléotidique au sein du sérotype 0157 :H7.

En effet, un produit amplifié de 670 paires de bases a été observé chez toutes les souches testées (55 *E. coli* O157 :H7 et 1 *E . coli* O157 :H-) avec les amorces SEQ ID N°3 et SEQ ID N°4, situées respectivement dans les séquences IS*91* et *katP.*

Par ailleurs, les données obtenues à partir des profils de restriction *Alu*I et *Rsa*I effectués sur les produits amplifiés de 5 souches O157 :H7 d'origines différentes, ainsi que l'analyse de séquence réalisée chez 3 souches, dont 2 isolées d'épidémies (USA, 1993 et Japon, 1996) ont montré une parfaite conservation c'est à dire 100 % d'homologie dans la partie de séquence analysée (SEQ ID N° 1 : positions (nt) 272 à 624).

De plus, l'enchaînement nucléotidique, stable et conservé, est probablement un événement de recombinaison relativement ancien, survenu chez *E*. *coli* 0157 :H7 au cours de l'évolution, puisque des souches isolées en des lieux et des époques différents présentent cette même caractéristique.

Cette séquence représente donc une cible de choix pour la détection spécifique du sérotype 0157 :H7.

Une seconde séquence a été caractérisée (SEQ ID N°2) sur le même plasmide, associée à la présence des facteurs de virulence entérohémolysine *(ehly)* et intimine *(eae),* caractères propres aux souches de *E*. *coli* entérohémorragiques, comprenant le sérotype O157 :H7. A ce titre, ce fragment présente un intérêt épidémiologique, car, à la différence des procédés déjà connus, nécessitant la mise en oeuvre de plusieurs systèmes moléculaires *(*Paton et Paton, J. Clin. Microbiol. 36, 1998, 598-602), l'utilisation de cette séquence pour une application diagnostique donne lieu à une mise en oeuvre simplifiée et à une interprétation plus rapide des résultats.

La présente invention a donc pour objet les séquences nucléiques SEQ ID N°1 et SEQ ID N°2 et leurs séquences complémentaires, les séquences dérivées de celles-ci et les fragments telles que définies ci-après, utilisables pour la détection spécifique des EHEC dans un échantillon alimentaire, clinique ou vétérinaire, ou de l'environnement.

La présente invention a plus particulièrement pour objet une séquence spécifique pour la détection du sérotype *E. coli* O157 :H7, comprenant la séquence SEQ ID N°1, un fragment de cette séquence ou une séquence dérivée de celle-ci, le fragment ou la séquence dérivée étant tels que définis ci-après.

Selon l'invention, la séquence SEQ ID N°1 comprend un enchaînement nucléotidique résultant d'un événement de recombinaison stable entre la séquence du gène *katP* ou une partie de celle-ci et une séquence d'insertion *IS*91 tronquée.

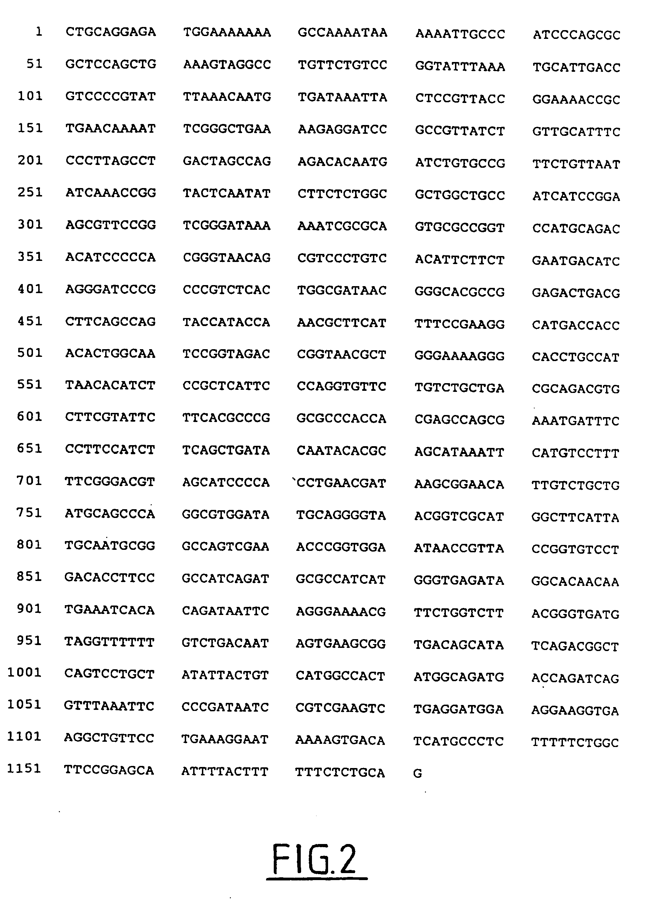

La présente invention a également pour objet une seconde séquence spécifique des EHEC, qui est la SEQ ID N°2, les séquences complémentaires de celles-ci, les fragments de celles-ci et les séquences dérivées de celles-ci, les fragments ou séquences dérivées étant tel(s) que défini(s) ci-après, ces séquences étant toujours détectées chez les EHEC, notamment chez les EHEC possédant conjointement les gènes codant pour l'entérohémolysine *(ehly)* et l'intimine *(eae)* ; ladite séquence SEQ ID N°2 étant représentée à la Figure 2.

Selon la présente invention, on entend par séquence nucléique aussi bien la séquence d'ADN ou d'ADN complémentaire (ADNc), ou encore la séquence d'ARN correspondante.

La demande décrit aussi des séquences différant de SEQ ID N°1 ou SEQ ID N°2 par mutation, insertion, délétion et/ou substitution d'une ou plusieurs bases mais s'hybridant néanmoins, dans des conditions de forte stringence, avec l'une des susdites séquences.

Des conditions de forte stringence sont des conditions de température et de force ionique telles qu'elles permettent une hybridation spécifique entre deux fragments d'acides nucléiques complémentaires et limitent les fixations spécifiques *(*Sambrook et al., Molecular Cloning, Second Edition (1989), 9.47-9.62*).* Les conditions de température sont généralement comprises entre (Tₘ moins 5°C) et (Tₘ moins 10°C) lorsque l'une des séquences de l'hybride est courte (une vingtaine de nucléotides), Tₘ étant la température théorique de fusion, définie comme étant la température à laquelle 50 % des brins appariés se séparent.

On entend par séquence nucléique dérivée de la SEQ ID N° 1, toute séquence différant de celle-ci par délétion et/ou substitution d'une ou plusieurs bases et comportant un enchaînement nucléotidique résultant de l'association stable d'au moins une partie de la séquence d'insertion *IS*91 et au moins une partie de la séquence du gène *kat*P, et s'hybridant dans des conditions de forte stingence avec la séquence SEQ ID N°1.

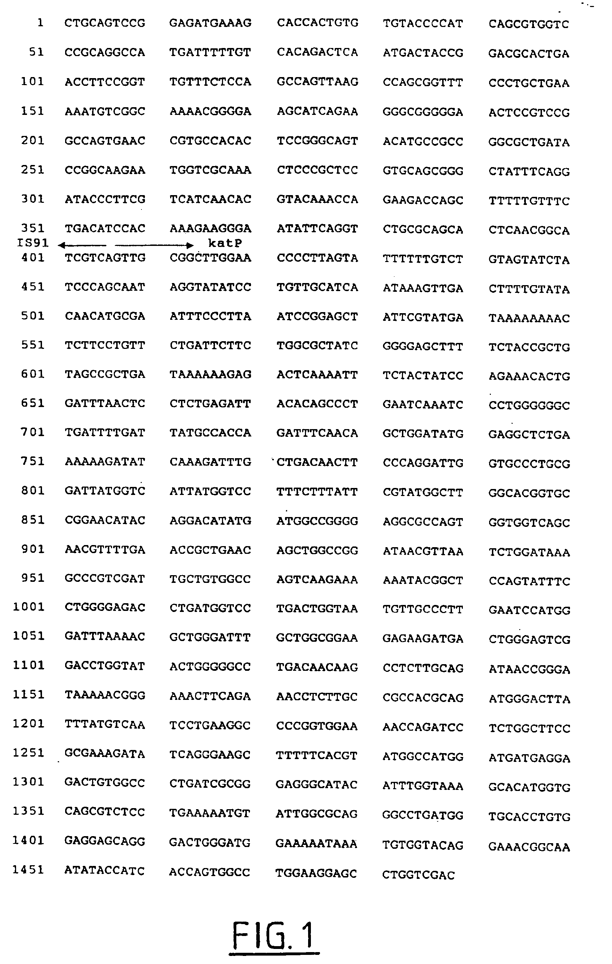

Plus particulièrement, les séquences nucléiques contiennent au moins 8 nucléotides, préférentiellement 10 nucléotides, ou très préférentiellement 14 nucléotides consécutifs de l'enchaînement de la Figure 1, et comprennent les nucléotides de la position 400 à la position 407.

On entend par séquence nucléique dérivée de la SEQ ID N°2 toute séquence différant de celle-ci par mutation, insertion, délétion et/ou substitution d'une ou plusieurs bases et s'hybridant dans des conditions de forte stringence avec la séquence SEQ ID N°2.

L'invention se rapporte également à des fragments oligonucléotidiques, issus des de la séquences SEQ ID N°2, utilisables comme amorces dans un processus d'amplification ou comme sonde dans le cadre de la mise en oeuvre d'un procédé de détection, comprenant au moins 8, avantageusement au moins 10, plus avantageusement 14 nucléotides, et de préférence jusqu'à 30 nucléotides consécutifs de l'enchaînement nucléotidique de SEQ ID N°2, lesdites amorces étant susceptibles de s'hybrider auxdites séquences dans des conditions de forte stringence, telles que définies ci-dessus.

L'invention se rapporte également à des fragments oligonucléotidiques, issus de la séquence SEQ ID N°1, utilisables comme amorces dans un processus d'amplification ou comme sonde dans le cadre de la mise en oeuvre d'un procédé de détection, comprenant au moins 14 nucléotides, et de préférence jusqu'à 30 nucléotides consécutifs de l'enchaînement nucléotidique de SEQ ID N°1, lesdits fragments comprenant un enchaînement nucléotidique résultant de l'association stable d'au moins une partie de la séquence d'insertion *IS*91 et au moins une séquence du gène *kat*P, et lesdites amorces étant susceptibles de s'hybrider auxdites séquences dans des conditions de forte stringence, telles que définies ci-dessus.

Les amorces ou sondes de l'invention comprennent également des oligonucléotides pouvant être modifiés par substitution et/ou addition et/ou suppression de plusieurs nucléotides, ou par l'addition à l'une des extrémités (généralement en 5' pour les amorces; 3' ou 5' pour les sondes) d'une séquence nucléique étrangère à la séquence recherchée, ou encore d'une molécule de marquage, lesdits oligonucléotides étant néanmoins capables de s'hybrider dans des conditions de forte stringence avec des séquences nucléiques complémentaires présentes chez E. *coli* O157 :H7 ou chez les EHEC.Selon un mode préféré de l'invention, les oligonucléotides peuvent être utilisés comme amorces, dans un processus d'amplification génique, conduisant à l'obtention d'une quantité importante de copies d'un fragment de SEQ ID N° 1 ou d'un fragment de SEQ ID N° 2 et permettant respectivement, la détection spécifique de E. *coli* O157 :H7 ou des EHEC.

L'étape d'amplification peut être réalisée par toute méthode utilisant les techniques classiques d'amplification enzymatique de l'ADN ou de l'ARN, telles que notamment, la technique TAS (Transcription-based Amplication System) proposée par Kwoh et al. dans PNAS, 86, 1989, 1173-1177, la technique 3SR (Self-Sustained Sequences Replication) décrite par Fahy et al. dans PCR Meth Appl. 1, 1991, 25-33*,* la technique NASBA (Nucleic Acid Sequence-Based Amplification) décrite dans le brevet EP 329 822, ou encore la technique SDA (Strand Displacement Amplification) décrite par Walker et al. dans P.N.A.S, 89, 1992, 392-396, ou avantageusement la technique PCR telle que décrite notamment dans les brevets Européens, EP 200 362 et EP 201 184, délivrés au nom de Cetus, ou encore les techniques dérivées de ces dernières et toute méthode visant à amplifier *in vitro* les séquences nucléiques.

Dans un mode de réalisation préféré de l'invention, les oligonucléotides issus des séquences SEQ ID N°1 et SEQ ID N°2 sont utilisés en PCR.

La détection des produits amplifiés peut être réalisée par électrophorèse sur gel de tout ou partie du milieu réactionnel dans lequel l'amplification a été effectuée, notamment sur gel d'agarose ou de polyacrylamide, ou par électrophorèse capillaire ou par chromatographie. La visualisation d'une bande de fragments nucléiques localisée en un point spécifique du gel permet d'en apprécier la taille, l'intensité de cette bande pouvant être corrélée grossièrement au nombre de copies initiales de la cible à détecter dans l'échantillon.

Selon un autre mode de réalisation de l'invention, les oligonucléotides, tels que définis ci-dessus, peuvent être utilisés comme sondes dans un processus d'hybridation pour la détection directe d'une séquence nucléique cible ou après amplification pour la détection des produits amplifiés.

A titre d'illustration, les fragments nucléotidiques peuvent être marqués par un élément radioactif (par exemple ³²P, ³⁵S, ³H, ¹²⁵I ) ou par une molécule non radioactive notamment biotine, acétylamino-fluorène, fluorochrome, digoxigénine ou par une molécule enzymatique, ou un haptène. Des exemples de marquages non radioactifs de sondes sont décrits, par exemple, dans le brevet français de P. Kourilsky n° 78.10975*,* ou par M.S. Urdea et al., Nucleic Acids Symp. Ser., 24, 1991, 197-200*,* ou encore par R. Sanchez-Pescador, J. Clin. Microbiol. 26, 1988. 1934-1938*.*

La méthode d'hybridation la plus générale consiste à immobiliser l'acide nucléique extrait de l'échantillon à analyser sur un support (nitro-cellulose, nylon polystyrène, etc. ) et à incuber dans des conditions définies de température et de force ionique, l'acide nucléique immobilisé avec la sonde. Après hybridation, l'excès de sonde est éliminé et les molécules hybrides formées sont détectées par la méthode appropriée (mesure de la radioactivité, de la fluorescence ou de l'activité enzymatique liée à la sonde).

Les molécules hybrides formées peuvent également être détectées sans qu'il soit nécessaire de séparer les phases « liée » et « non liée ». On dit alors que la détection s'effectue en phase homogène. Ces méthodes, telles que décrites par T. Walker et al. Clin. Chemistry 42, 1996, 9-13 et L. Morrison (Nonisotopic DNA Probe Techniques, Academic Press, 1992, 312-352*)* concernent notamment la polarisation de fluorescence, dans laquelle une sonde est marquée à la fluoresceine et où l'hybridation entraîne une modification de la fluorescence, ou encore le transfert d'énergie. Dans ce dernier cas, la détection repose sur des interactions inter ou intramoléculaires entre deux marqueurs. Un premier marqueur appelé « donneur » est excité par absorption d'une lumière à une longueur d'onde particulière. L'énergie est transférée à un second marqueur appelé « accepteur », qui est à son tour excité et émet de l'énergie.

Les sondes oligonucléotidiques peuvent également être mises en oeuvre au sein d'un dispositif de détection comprenant un arrangement matriciel d'oligonucléotides dans lequel, des oligonucléotides d'une longueur donnée, sont fixés dans un ordre prédéterminé sur un support et se chevauchent les uns par rapport aux autres d'une ou plusieurs bases ; chaque oligonucléotide étant complémentaire d'une séquence d'ADN ou d'ARN de la séquence cible à détecter. La séquence cible, avantageusement marquée est mise en contact avec le dispositif matriciel et peut s'hybrider aux sondes fixées sur le support. Un traitement enzymatique permet ensuite d'éliminer les hybrides incomplets. Connaissant la séquence d'une sonde à une position déterminée de la matrice, il est ainsi possible de déduire la séquence nucléotidique de la séquence cible analysée et de déduire les mutations éventuellement survenues.

Une alternative à l'utilisation d'une séquence marquée peut consister en l'utilisation d'un support permettant une détection « bioélectronique » de l'hybridation de la séquence cible sur les sondes fixées sur le support d'un matériau, tel que l'or, capable d'agir, par exemple, en tant que donneur d'électrons aux positions de la matrice auxquelles un hybride est formé. La détection de la séquence nucléique cible est alors déterminée par un dispositif électronique. Un exemple de réalisation d'un biocapteur est décrit dans la demande de brevet EP-0721 016 au nom d'Affymax Technologies.

Selon un mode simple et avantageux de mise en oeuvre, les sondes nucléiques peuvent être utilisées comme sondes de capture. Dans ce cas, la sonde dite « sonde de capture » est immobilisée sur un support et sert à capturer par hybridation spécifique l'acide nucléique cible à partir de l'échantillon à tester. Si nécessaire, le support solide est séparé de l'échantillon et le duplex formé entre la sonde de capture et la séquence d'acide nucléique cible est ensuite détecté grâce à une seconde sonde dite « sonde de détection », marquée par un élément détectable. Avantageusement, les sondes de capture et de détection sont complémentaires de deux régions différentes comprises dans la séquence cible (amplifiée ou non ) à détecter.

La fixation de la sonde de capture sur le support solide peut se faire selon des procédés bien connus de l'homme du métier, notamment par adsorption passive ou par couplage covalent *(*Cook et al., Nucleic Acids Res. 16, 1988, 4077-4095 *;* Nagata et al., FEBS Lett. 183, 1985, 379-382 *;* M. Longlaru et al., EP 420 260 A2 *;* T. Gingeras et al., EP 276 302 *;* E. Hornes et LM. Kornes, EP 446 260*).*

L'hybridation des sondes de capture et de détection peut se faire séparément (en deux temps) ou simultanément (en un temps), notamment selon l'une des méthodes décrites par Langhale et Malcolm, Gene 36, 1985, 201-210 ou par Ranki et al, Gene 21, 1993, 77-85. par Dunn et Hassel, Cell, 12, 1977, 23-36 ou encore par Ranki et Soderlund dans les brevets US 4,486,539 et US 4,563,419.

Des oligonucléotides issus de SEQ ID N°1 ou de SEQ ID N°2, sélectionnés comme amorces ou comme sondes, sont susceptibles de s'hybrider dans des conditions stringentes avec une séquence d'acide nucléique cible contenue dans l'échantillon testé spécifiques de *E*. *coli* 0157 :H7 ou des EHEC.

On entend par séquence nucléique cible, toute molécule d'ADN ou ADNc ou d'ARN capable de s'hybrider dans des conditions de forte stringence avec un oligonucléotide selon l'invention.

Les oligonucléotides préférés dont les séquences sont spécifiées en annexe correspondent aux positions sur les séquences SEQ ID N° 1 et SEQ ID N° 2 rapportées dans le tableau ci-après :

| Séquence | Position dans SEQ ID N°1 |
|---|---|
| SEQ ID N°3 : | 9 - 30 |
| SEQ ID N°4 : | 679 - 658 |
| SEQ ID N°5: | 6 - 30 |
| SEQ ID N°6 : | 682 - 658 |
| SEQ ID N°7 : | 241 - 263 |
| SEQ ID N°8 : | 47 - 69 |
| SEQ ID N°9 : | 251 - 274 |
| SEQ ID N°10 : | 426 - 401 |
| SEQ ID N°11 : | 427 - 402 |
| SEQ ID N°12 : | 391 - 421 |
| SEQ ID N°13 : | 387 - 417 |
| SEQ ID N°14 : | 291 - 321 |
| SEQ ID N°15 : | 510 - 540 |
| SEQ ID N°16 : | 331 - 350 |
| SEQ ID N°17 : | 68 - 87 |
| SEQ ID N°18 : | 397 - 410 |
| SEQ ID N°19 : | 396 - 411 |
| SEQ ID N°20 : | 395 - 412 |

| | SEQ ID N°2 |
|---|---|
| SEQ ID N°21 : | 718 - 739 |
| SEQ ID N°22 : | 1099 - 1078 |
| SEQ ID N°23 : | 41 - 60 |
| SEQ ID N°24 : | 884 - 863 |
| SEQ ID N°25 : | 928 - 958 |
| SEQ ID N°26 : | 970 - 1000 |
| SEQ ID N°27 : | 883 - 903 |

L'invention concerne aussi des couples d'oligonucléotides, tels que décrits précédemment, susceptibles d'être utilisés comme amorces pour l'amplification d'une séquence nucléique cible correspondant à SEQ ID N°1 ou SEQ ID N°2, contenue dans le génome de *E*. *coli* O157 :H7 ou des EHEC :
Les couples d'amorces préférées sont les suivants :
   - pour l'amplification de *E. coli* O157 :H7
      - SEQ ID N° 3 et SEQ ID N° 4
      - SEQ ID N° 5 et SEQ ID N° 6
      - SEQ ID N° 6 et SEQ ID N° 7
      - SEQ ID N° 6 et SEQ ID N° 8
      - SEQ ID N° 6 et SEQ ID N° 9
   - pour l'amplification des EHEC :
      - SEQ ID N° 21 et SEQ ID N° 22
      - SEQ ID N° 23 et SEQ ID N° 24

Ainsi, l'utilisation du couple d'amorces SEQ ID N°5 et SEQ ID N°6 pour réaliser l'amplification de l'acide nucléique de *E*. *coli* 0157 :H7 conduit à l'amplification d'un fragment nucléique de 676 pb, caractéristique des souches de *E. coli* 0157 :H7. La spécificité de ce fragment peut être éventuellement contrôlée par l'utilisation de la sonde SEQ ID N° 18.

De même, l'utilisation du couple d'amorces SEQ ID N° 21 et SEQ ID N° 22 amplifie spécifiquement une séquence nucléique de 382 pb présente chez les EHEC, possédant aussi les caractères entérohémolysine et intimine. Les bactéries appartenant aux autres groupes de *E*. *coli,* comme les ETEC (E. *coli* producteurs d'entérotoxines), les EPEC etc, ne sont pas détectées. La spécificité du produit amplifié peut être par ailleurs confirmée à l'aide de sondes oligonucléotidiques internes au fragment amplifié, telles que SEQ ID N°27.

La présente invention a également pour objet des oligonucléotides, tels que précédemment décrits, susceptibles d'être utilisés comme sondes pour la détection d'une séquence de nucléotides, éventuellement amplifiée. Par exemple, les séquences oligonucléotidiques SEQ ID N° 14, SEQ ID N°15 et SEQ ID N° 18 peuvent être utilisées pour la détection spécifique de *E. coli* 0157 :H7. De même, l'utilisation des séquences SEQ ID N° 25, SEQ ID N° 26 et SEQ ID N° 27 permet la détection des EHEC dont 0157:H7.

L'invention a également pour objet les plasmides contenant les séquences SEQ ID N° 1 et SEQ ID N° 2 mentionnées précédemment ainsi que les cellules hôtes les contenant.

L'invention vise aussi un procédé pour la détection *in vitro* de *E*. *coli* 0157 :H7 ou des EHEC dans un échantillon, **caractérisé en ce qu**'il comprend les étapes suivantes :
1. la mise en contact de l'échantillon avec l'un des couples d'amorces, tels que décrits ci-dessus, l'acide nucléique contenu dans l'échantillon ayant été, le cas échéant, rendu accessible à l'hybridation des amorces à l'acide nucléique de la cible recherchée,
2. l'amplification de la séquence nucléique encadrée par le couple d'amorces choisi,
3. la vérification de la présence éventuelle du produit amplifié pouvant s'effectuer selon une méthode connue de l'homme du métier, telle que précédemment décrite.

Selon un mode de réalisation avantageux, les fragments amplifiés peuvent être détectés selon le principe de la méthode dite « sandwich ».

Entre également dans le cadre de l'invention, un procédé pour la détection *in vitro* de séquences nucléotidiques spécifiques de *E. coli* 0157 : H7 ou des EHEC, préalablement amplifiées par détection sur un support, par exemple une plaque de micro-titration et, **caractérisé en ce qu**'il comprend les étapes suivantes :
- dénaturation de la séquence amplifiée de *E. coli* O157 :H7 et/ou des EHEC par un moyen physique ou chimique. On préférera l'addition d'une solution dénaturante composée de 200 mM NaOH, 40 mM EDTA,
- mise en contact, dans un tampon d'hybridation approprié des fragments amplifiés dénaturés avec, d'une part au moins une sonde de capture fixée sur le support, et d'autre part, au moins une sonde nucléique de détection libre dans le tampon d'hybridation, éventuellement marquée, susceptible de s'hybrider avec le même brin des fragments amplifiés que celui avec lequel la sonde de capture est hybridée, mais en une région distincte de celle hybridée avec la sonde de capture ; ladite solution d'hybridation pouvant être avantageusement SSPE *(*Sodium Saline Phosphate EDTA; Molecular Cloning, A practical guide, Sambrook et al., Vol.3, 1989, annexe B13*)* 5 fois concentré, 0,5% Tween 20, Merthiolate 0,01 % ,
- incubation du mélange réactionnel pendant une période suffisamment longue pour permettre l'hybridation ; cette incubation pouvant être, par exemple avantageusement accomplie à 37°C pendant environ 1 heure,
- un ou plusieurs lavage(s) du mélange précédent, afin d'éliminer toute séquence nucléique n'ayant pas réagi ; lesdits lavages pouvant être par exemple effectués avec une solution contenant du Tris-HCl 10 mM, NaCl 300 mM et Tween 20 0,1%, pH 7,4.
- révélation des sondes de détection hybridées aux séquences nucléiques amplifiées.

Selon un mode avantageux de l'invention, la sonde de détection est marquée à la péroxydase, et la révélation de l'activité de la péroxydase liée à la sonde de détection hybridée est réalisée par lecture colorimétrique, en présence d'un substrat chromogène, selon les étapes suivantes :
- dépôt d'une solution contenant un substrat chromogène, tel que le tetraméthylbenzidine (TMB ), dans chacun des puits contenant le mélange de la réaction, et incubation à l'obscurité de la microplaque pendant un temps suffisant, généralement 20 à 30 min., puis la réaction est arrêtée par l'addition d'une solution d'arrêt, ladite solution étant avantageusement une solution de H₂SO₄ utilisée à une concentration finale de 0.5 N, :
- détermination de la densité optique, ladite détermination étant réalisée à une longueur d'onde de 450 nm (référence 620 nm) lorsque le TMB est utilisé comme substrat chromogène.

Selon un mode de réalisation particulièrement avantageux, la sonde de capture utilisée pour la détection de *E*. *coli* 0157 :H7 peut être SEQ ID N°15 et la sonde de détection est l'oligonucléotide SEQ ID N°18. De même, la sonde de capture utilisée pour la détection des bactéries EHEC peut être SEQ ID N°25 et la sonde de détection l'oligonucléotide SEQ ID N°27.

L'invention concerne également une trousse de détection, pour l'identification de *E. coli* O157 :H7 ou des EHEC, contenus dans un échantillon, comprenant parmi les réactifs:
- au moins deux oligonucléotides sélectionnés dans le groupe consistant en SEQ ID N°3 à SEQ ID N°20, utilisés comme amorces pour l'amplification de *E. coli* O157 :H7 ou au moins deux oligonucléotides sélectionnés dans le groupe consistant en SEQ ID N°21 à SEQ ID N°27 utilisés comme amorces pour l'amplification des bactéries du groupe EHEC,
- éventuellement, un composant pour vérifier la séquence du fragment amplifié, plus particulièrement, une sonde nucléique sélectionnée dans le groupe consistant en SEQ ID N°3 à SEQ ID N°20 (*E. coli* O157 :H7) ou SEQ ID N°21 à SEQ ID N°27 (EHEC).

Les exemples suivants sont donnés à titre non limitatif pour illustrer l'invention.

### EXEMPLE 1

### Caractérisation des séquences SEQ ID N°1 et SEQ ID N°2

### 1) Construction de la banque génomique de E. coli 0157 :H7 :

L'ADN génomique de la souche *E. coli* O157 :H7 isolée des selles d'un patient atteint de colite hémorragique et productrice des vérotoxines de type 1 et 2 a été digéré partiellement par l'endonucléase *Pstl* (Boehringer Mannheim; Réf. 621625) en faisant agir 0.03 unité d'enzyme par µg d'ADN en milieu tamponné pendant 1 heure à 37°C. L'ADN génomique ainsi digéré a permis de générer des fragments de 35-45 kb. Le cosmide pHC79 *(*Hohn et Murray, Proc. Natl. Acad. Sci 74, 1977, 3259-3263*)* a été digéré de la même manière et déphosphorilé pour éviter toute autoligation.

La ligation s'est effectuée en mélangeant 900 ng de vecteur et 2.6 µg de fragments d'ADN de 35-45 kb (soit un rapport molaire vecteur/insert de 2), le milieu réactionnel étant laissé à 14°C pendant 18 heures après avoir été additionné de 2 unités de T4 DNA ligase (Boehringer Mannheim ; Réf. 481220). Les cosmides recombinants ont été encapsidés *in vitro* et utilisés pour transformer les bactéries *E*. *coli* XL1-Blue MR (Stratagene ; Réf. 200300). Les bactéries transformées ont été incubées 1 heure à 37°C en milieu LB *(*Luria-Bertani, Molecular Cloning, A practical guide, Sambrook et al., Vol.3, 1989, annexe A1*).* Les fragments d'ADN de 35-45 kb étant insérés dans le vecteur pHC79 de manière à abolir le site de résistance ampicilline et préserver le site de résistance tétracycline, les bactéries ont été ensuite étalées sur milieu sélectif gélosé contenant 12.5 µg/ml de tétracycline.

Des mini-préparations d'ADN cosmidique ont été effectuées à partir des 360 premières colonies isolées sur tétracycline en utilisant le Kit REAL Prep96 distribué par Quiagen (Réf. 26171).

L'ADN de ces préparations a été ensuite digéré par les endonucléases *Pstl, EcoRI* et *Sall* (Boehringer Mannheim, Réf. 621625, 703737 et 567663) analysé en électrophorèse sur gel d'agarose à 1.2 % puis transféré sur filtre de nylon Hybond N⁺ (Amersham, Réf. RPN 303B). L'ADN a été fixé de façon irréversible par 5 mn d'exposition aux UV.

### 2) Criblage de la banque :

Les hybridations ont été réalisées avec une sonde d'ADN homologue provenant de la souche *E. coli* 0157 :H7 (Collection de l'Institut Pasteur, n° 103571) et avec une sonde d'ADN hétérologue constituée d'un « pool » d'ADN provenant de 8 souches *E*. *coli* O55 :H7 (Collection de l'Institut Pasteur, n° 105215, 105216, 105217, 105228, 105239, 105240, 105241, 105242).

Les différents filtres ont été hybridés pendant 16 à 18 heures à 65°C dans une solution contenant du tampon SSC *(*Sodium Saline Citrate ; Molecular Cloning, A practical guide, Sambrook et al., Vol. 3, 1989, annexe B13*)* 6 fois concentré, de la solution de Denhart 5 fois concentrée *(*Molecular Cloning, Vol. 3, 1989, annexe B15*),* 10 % de sulfate dextran (Pharmacia Biotech, Réf. 17-0340-02), 10 mM EDTA, 0.5 % SDS, 100 µg/ml ADN simple brin de sperme de saumon et l'ADN relevant (0157 :H7).

Après hybridation, les filtres ont été lavés 2 fois 10 mn dans un tampon SSC 2 fois concentré à 65°C, 1 fois 30 mn dans un tampon contenant du SSC 2 fois concentré et 0.1 % SDS à 65°C puis 1 fois 10 mn dans du SSC dilué au 1/10è à 65°C. Les filtres encore humides ont été exposés en cassette avec un écran intensifiant pendant 24 à 48 heures à -80°C.

Après le temps d'exposition nécessaire, les films ont été développés puis les membranes de nylon déshybridées en effectuant 4 à 5 cycles de bains à 45°C sous agitation. Pour chaque cycle, 2 bains successifs de 30 mn dans une solution de NaOH 0.5 N puis 30 mn dans un tampon contenant du SSC dilué au 1/10è et SDS 0.1 % ont été effectués. Les membranes ont finalement été lavées en SSC 2 fois concentré et mises en cassette pour vérifier qu'il ne reste plus de traces d'hybridation. Après déshybridation, les filtres ont été hybridés de la même manière que précédemment avec un pool d'ADN non relevant (055 :H7).

### 3) Isolement et clonage des fragments SEQ ID N°1 et SEQ ID N°2 :

Les résultats de ces hybridations ont permis d'identifier deux clones cosmidiques à partir desquels un fragment d'environ 1 à 2 kb, hybridant avec la sonde homologue et n'hybridant pas avec la sonde hétérologue, a été isolé respectivement. Après avoir vérifié leur conservation chez différentes souches O157 :H7 par hybridation en « dot-blot », ces fragments ont été clonés dans un vecteur pUC18 (Oncor Appligene, Réf. 161131) puis préparés en grande quantité. Les plasmides recombinants ont été dénommés pDF3 et pDF4 et correspondent respectivement aux séquences SEQ ID N°1 et SEQ ID N°2.

### 4) Détermination des séquences SEQ ID N°1 et SEQ ID N°2

Les fragments ont été séquencés selon la méthode de Sanger et al. décrite dans Proc. Natl. Acad. Sci. 74, 1977, 5463*,* en utilisant le « primer universel » et le « reverse primer » du plasmide pUC18, ainsi que des oligonucléotides internes aux séquences.

La séquence SEQ ID N°1 (Figure 1), contenant 1489 pb, présente une homologie de 99,9 % avec le gène *katP* de *E*. *coli* 0157 :H7 dans la région 407 à 1489 et une homologie de 95,8 % avec l'IS*91* de *E*. *coli* dans la région 1 à 406.

L'analyse de la séquence SEQ ID N°2 (Figure 2), contenant 1181 pb, ne révèle aucune séquence connue du plasmide entérohémolytique. Seule la partie 237 à 570 présente une homologie de 68 % avec le gène plasmidique *vir*K. codant pour une protéine de virulence de *Shigella flexneri.*

### EXEMPLE 2

### Détection spécifigue de E. coli O157 :H7

L'étude de spécificité a été effectuée sur 100 souches de *E*. *coli* de sérotypes différents et 42 souches non *E*. *coli* comprenant, entre autres, des bactéries susceptibles de présenter des réactions croisées avec *E*. *coli* 0157 :H7 comme par exemple *Salmonella, Shigella dysenteriae, Citrobacter freundii, Hafnia alvei, Escherichia hermanii.*

### 1) Extraction de l'ADN :

On obtient les séquences d'ADN par la méthode d'ébullition en présence de Chelex (InstaGene^{™} Matrix, Biorad). Les échantillons ont été préparés selon le protocole suivant :

Une suspension bactérienne est effectuée dans de l'eau ultra pure stérile à partir de plusieurs colonies bactériennes isolées sur gélose Tryptone-Caséine-Soja (Sanofi Diagnostics Pasteur, Réf. 53455), puis centrifugée à 10000-12000 tours / min pendant 2-3 min et le surnageant soigneusement éliminé. Le culot bactérien est resuspendu dans 200 µl de réactif de lyse, homogénéisé puis incubé dans un bloc chauffant à 100°C pendant 10-15 min. L'échantillon est de nouveau homogénéisé, puis centrifugé à 10000-12000 tours / min pendant 2-3 min. L'ADN peut être amplifié directement ou stocké à -20°C.

### 2) Amplification par PCR :

La réaction d'amplification est réalisée dans un volume total de 50 µl contenant 50 mM KCl; 10 mM Tris-HCl pH 8,3 ; 0,01% gélatine; 3 mM MgCl₂: 0,25 µM de chaque amorce SEQ ID N°5 et SEQ ID N°6 ; 100 µM (dATP, dCTP. dGTP) ; 400 µM dUTP ; 0,5 unité d'Uracyl-DNA-Glycosylase (UDG ; BRL Life Technologies) ; 1 unité de Taq DNA Polymérase (BRL Life Technologies) et 5 µl d'ADN préparé comme indiqué dans le paragraphe 1.

Après une incubation à 50°C pendant 2 min puis à 95°C pendant 5 min, les échantillons sont soumis à 35 cycles d'amplification composés de 15 sec à 95°C, 15 sec à 65 °C et 15 sec à 72°C. Les tubes sont maintenus à 72°C jusqu'au retrait du plateau.

Les cycles thermiques sont réalisés dans un thermocycleur « Perkin-Elmer 9600 ».

Chaque expérience comprend un contrôle positif et un contrôle négatif.

### 3) Visualisation des produits amplifiés :

Les réactions d'amplification sont visualisées sur gel d'agarose ou détectées sur microplaque.

### 3-1) Gel d'agarose :

Après amplification, 50 µl de chloroforme sont ajoutés à chaque échantillon afin d'inactiver l'UDG puis une aliquote de chaque réaction est analysée par électrophorèse sur gel d'agarose à 1,2 % coloré au bromure d'éthidium, en présence d'un marqueur de taille. La visualisation d'un fragment d'ADN à 676 pb indique la présence de *E*. *coli* 0157 :H7 dans l'échantillon testé.

### 3-2) Hybridation en microplaque :

Les produits d'amplification sont dénaturés par addition volume à volume d'une solution contenant 200 mM NaOH, 40 mM EDTA. La microplaque dont la surface des puits est revêtue de la sonde de capture SEQ ID N°15 est préhybridée dans un tampon d'hybridation contenant du SSPE 5 fois concentré. 0,5 % Tween 20 et 0,01 % Merthiolate. Puis la microplaque est vidée et chacune des cupules reçoit 200 µl de tampon d'hybridation contenant le fragment amplifié dénaturé et la sonde de révélation SEQ ID N°18. L'incubation est effectuée à 37°C sous agitation pendant 1 heure.

Les cupules sont ensuite lavées six fois avec 400 µl de solution (10 mM Tris-HCl pH 7,4; 300 mM NaCl et 0,1% Tween 20), puis l'activité de la péroxydase liée à la sonde est détectée en ajoutant dans chaque cupule 200 µl d'une solution de détection contenant le chromogène tétraméthylbenzidine (TMB). La microplaque est incubée à 37°C dans l'obscurité pendant 30 min puis 100 µl d'une solution de 1,5 N H₂SO₄ sont ajoutés pour bloquer les réactions. Les densités optiques sont déterminées à 450 nm contre une référence à 620 nm.

### 4) Etude de la spécificité :

Les tests ont été effectués sur un total de 142 souches bactériennes, utilisant le couple d'amorces SEQ ID N°5 et SEQ ID N°6 pour l'étape d'amplification par PCR, la sonde de capture SEQ ID N°15 et la sonde de détection SEQ ID N°18 pour l'étape d'hybridation sur microplaque.

Les résultats obtenus sur microplaque avec les souches *E*. *coli* et non *E. coli* (bactéries de genres et espèces différents) sont présentés respectivement dans les Tableaux I et II ci-dessous :

**Tableau I**

| **Souche E. coli (sérotype)** | **Nombre de souches testées** | **PCR SEQ ID N° 5 / 6** |
|---|---|---|
| | | |
| **VTEC/EHEC** | | |
| O157:H7 | 55 | + |
| O157:H- | 1 | + |
| | | |
| O26:H11 | 10 | - |
| O111:H- | 2 | - |
| O145:H- | 2 | - |
| O103:H2 | 2 | - |
| O121:H19 | 1 | - |
| O165:H25 | 1 | - |
| O45:H2 | 1 | - |
| O22:H8 | 2 | - |
| O137:H41 | 1 | - |
| O91:H21 | 1 | - |
| O141:H4 | 1 | - |
| | | |
| **EPEC** | | |
| O55:H7 | 8 | - |
| O55:H6 | 1 | - |
| O55:H- | 1 | - |
| O111:H- | 1 | - |
| O111:H2 | 1 | - |
| O111:H12 | 1 | - |
| O128:H2 | 1 | - |
| O127:H6 | 1 | - |
| | | |
| **ETEC** | | |
| O157:H19 | 1 | - |
| O159:H34 | 1 | - |
| CIP 81.86 | 1 | - |
| | | |
| **E. coli** | | |
| CIP 76.24 | 1 | - |
| CIP 54.8 | 1 | - |
| | | |

| | | |
|---|---|---|
| Les résultats (+) correspondent à DO₄₅₀ > 2,5. Les résultats (-) correspondent à DO₄₅₀ < 0,05. | | |

**Tableau II**

| **Souche** **(espèce bactérienne)** | **Nombre de souches testées** | **PCR SEQ ID N° 5 / 6** |
|---|---|---|
| | | |
| **Salmonella** | | |
| Salmonella (groupes I à VI) | 10 | - |
| | | |
| **Shigella** | | |
| Shigella flexneri | 2 | - |
| Shigella dysenteriae | 1 | - |
| Shigella sonnei | 1 | - |
| | | |
| **Autres** | | |
| Escherichia hermanii | 2 | - |
| Citrobacter freundii | 2 | - |
| Yersinia enterocolitica | 2 | - |
| Yersinia pseudotuberculosis | 1 | - |
| Hafnia alvei | 1 | - |
| Proteus mirabilis | 1 | - |
| Proteus vulgaris | 1 | - |
| Serratia marcescens | 1 | - |
| Klebsiella pneumoniae | 2 | - |
| Klebsiella oxytoca | 1 | - |
| Enterobacter cloacae | 1 | - |
| Enterobacter aerogenes | 1 | - |
| Enterobacter agglomerans | 1 | - |
| Bacillus subtilis | 1 | - |
| Morganella morganii | 1 | - |
| Providencia alcalifaciens | 1 | - |
| Vibrio parahaemolyticus | 1 | - |
| Acinetobacter baumanii | 1 | - |
| Shewanella putrefaciens | 1 | - |
| Pseudomonas aeruginosa | 1 | - |
| Pseudomonas fluorescens | 1 | - |
| Listeria monocytogenes | 3 | - |
| | | |

| | | |
|---|---|---|
| Les résultats (+) correspondent à DO₄₅₀ > 2,5. Les résultats (-) correspondent à DO₄₅₀ < 0,05. | | |

En conclusion, seules les souches 0157 :H7 et 0157 :H- sont détectées sur microplaque avec le système susmentionné.

### EXEMPLE 3

### Détection spécifique des EHEC

La spécificité a été testée sur un total de 142 souches bactériennes incluant différents sérotypes de *E. coli* ainsi que d'autres espèces bactériennes pouvant interférer avec la détection des EHEC.
Les ADN ont été extraits selon le protocole décrit dans le premier paragraphe de l'exemple 2.

Les conditions d'amplification sont les suivantes :
La réaction est réalisée dans un volume total de 50 µl contenant 50 mM KCl; 10 mM Tris-HCl pH 8,3 ; 1,5 mM MgCl₂; 0,5 µM de chaque amorce SEQ ID N°21 et SEQ ID N°22 ; 200 µM (dATP, dCTP, dGTP, dTTP) ; 1 unité de Taq DNA Polymérase (BRL Life Technologies) et 5 µl d'ADN préparé comme indiqué dans le paragraphe 1 de l'exemple 2.

Les cycles thermiques sont réalisés dans un thermocycleur « Perkin-Elmer 9600 ».

Chaque expérience comprend un contrôle positif et un contrôle négatif.

Les produits d'amplification ont été visualisés sur gel d'agarose coloré au BET, la présence d'une bande à 382 pb témoignant de la présence d'EHEC dans l'échantillon testé.

Les résultats sont présentés dans le Tableau III.

Seules les souches présentant les *caractères ehly* et eae, facteurs de virulence fréquemment associés chez les souches isolées d'infections humaines, sont détectées par PCR avec le couple d'amorces SEQ ID N°21 et SEQ ID N°22.

De plus, l'utilisation dudit couple d'amorces permet également de détecter en particulier, grâce à une réaction d'amplification unique, les souches de *E. coli* possédant le génotype (*vt*⁺, *eae*⁺ et *ehly*⁺), caractéristique des *E. coli* entérohémorragiques.

**Tableau III**

| **Souche** **(sérotype)** | **Nombre de souches testées** | **Génotype** | **PCR SEQ ID N°21/22** |
|---|---|---|---|
| **VTEC/EHEC** | | | |
| O157: H7 | 54 | vt+ , ehly+ , eae+ | + |
| | 1 | vt- , ehly+ , eae+ | + |
| O157:H- | 1 | vt+ , ehly+ , eae+ | + |
| O26:H11 | 5 | vt+ , ehly+ , eae+ | + |
| | 1 | vt- , ehly+ , eae+ | + |
| O26: H- | 1 | vt+ , ehly+ , eae+ | + |
| O111: H- | 1 | vt+ , ehly+ , eae+ | + |
| O145:H- | 2 | vt+, ehly+ , eae+ | + |
| O103:H2 | 2 | vt+ , ehly+ , eae+ | + |
| O121:H19 | 1 | vt+ , ehly+ , eae+ | + |
| O165: H25 | 1 | vt+ , ehly+ , eae+ | + |
| O45: H2 | 1 | vt+ , ehly+ , eae+ | + |
| | | | |
| O22: H8 | 2 | vt+ , ehly+ , eae- | - |
| O137:H41 | 1 | vt+ , ehly+ , eae- | - |
| O91:H21 | 1 | vt+ , ehly+ , eae- | - |
| | | | |
| O26:H11 | 3 | vt+ , ehly- , eae+ | - |
| O111:H- | 1 | vt+ , ehly- , eae+ | - |
| O141:H4 | 1 | vt+ , ehly- , eae- | - |
| | | | |
| **EPEC** | | | |
| O55:H7 | 8 | vt- , ehly- , eae+ | - |
| O55: H6 | 1 | vt- , ehly- , eae- | - |
| O55: H- | 1 | vt- , ehly- , eae- | - |
| O111: H- | 1 | vt- , ehly- , eae- | - |
| O111: H2 | 1 | vt- , ehly- , eae+ | - |
| O111:H12 | 1 | vt- , ehly- , eae- | - |
| O128:H2 | 1 | vt- , ehly- , eae+ | - |
| O127:H6 | 1 | vt- , ehly- , eae+ | - |
| | | | |
| **ETEC** | | | |
| O157:H19 | 1 | vt- , ehly- , eae- | - |
| O159:H34 | 1 | vt- , ehly- , eae- | - |
| CIP 81.86 | 1 | vt- , ehly- , eae- | - |
| | | | |
| **E. coli** | | | |
| CIP 76.24 | 1 | vt- , ehly- , eae- | - |
| CIP 54.8 | 1 | vt- , ehly- , eae- | - |
| | | | |
| **Non E. coli** | 42 | | - |

### LISTE DE SEQUENCES

(1) INFORMATION GENERALE
   (i) DEPOSANT:
      (A) NOM : PASTEUR SANOFI DIAGNOSTICS
      (B) RUE : 3 BOULEVARD RAYMOND POINCARE
      (C) VILLE : MARNES LA COQUETTE
      (D) PAYS : FRANCE
      (E) CODE POSTAL: 92430
   (ii) TITRE DE L'INVENTION : SEQUENCES NUCLEOTIDIQUES POUR LA DETECTION DES *ESCHERICHIA COLI* ENTEROHEMORRAGIQUES
   (iii) NOMBRE DE SEQUENCES : 27
(2) INFORMATION POUR LA SEQUENCE ID N°1:
   (i) CARACTERISTIQUE DE LA SEQUENCE :
      (A) LONGUEUR : 1489 paires de bases
      (B) TYPE : acide nucléique
      (C) NOMBRE DE BRINS : double
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°1 :
   1 CTGCAGTCCG GAGATGAAAG CACCACTGTG TGTACCCCAT CAGCGTGGTC
   51 CCGCAGGCCA TGATTTTTGT CACAGACTCA ATGACTACCG GACGCACTGA
   101 ACCTTCCGGT TGTTTCTCCA GCCAGTTAAG CCAGCGGTTT CCCTGCTGAA
   151 AAATGTCGGC AAAACGGGGA AGCATCAGAA GGGCGGGGGA ACTCCGTCCG
   201 GCCAGTGAAC CGTGCCACAC TCCGGGCAGT ACATGCCGCC GGCGCTGATA
   251 CCGGCAAGAA TGGTCGCAAA CTCCCGCTCC GTGCAGCGGG CTATTTCAGG
   301  ATACCCTTCG TCATCAACAC GTACAAACCA GAAGACCAGC TTTTTGTTTC
   351  TGACATCCAC AAAGAAGGGA ATATTCAGGT CTGCGCAGCA CTCAACGGCA
   401  TCGTCAGTTG CGGCTTGGAA CCCCTTAGTA TTTTTTGTCT GTAGTATCTA
   451  TCCCAGCAAT AGGTATATCC TGTTGCATCA ATAAAGTTGA CTTTTGTATA
   501  CAACATGCGA ATTTCCCTTA ATCCGGAGCT ATTCGTATGA TAAAAAAAAC
   551  TCTTCCTGTT CTGATTCTTC TGGCGCTATC GGGGAGCTTT TCTACCGCTG
   601  TAGCCGCTGA TAAAAAAGAG ACTCAAAATT TCTACTATCC AGAAACACTG
   651  GATTTAACTC CTCTGAGATT ACACAGCCCT GAATCAAATC CCTGGGGGGC
   701  TGATTTTGAT TATGCCACCA GATTTCAACA GCTGGATATG GAGGCTCTGA
   751  AAAAAGATAT CAAAGATTTG CTGACAACTT CCCAGGATTG GTGCCCTGCG
   801  GATTATGGTC ATTATGGTCC TTTCTTTATT CGTATGGCTT GGCACGGTGC
   851  CGGAACATAC AGGACATATG ATGGCCGGGG AGGCGCCAGT GGTGGTCAGC
   901  AACGTTTTGA ACCGCTGAAC AGCTGGCCGG ATAACGTTAA TCTGGATAAA
   951  GCCCGTCGAT TGCTGTGGCC AGTCAAGAAA AAATACGGCT CCAGTATTTC
   1001 CTGGGGAGAC CTGATGGTCC TGACTGGTAA TGTTGCCCTT GAATCCATGG
   1051 GATTTAAAAC GCTGGGATTT GCTGGCGGAA GAGAAGATGA CTGGGAGTCG
   1101 GACCTGGTAT ACTGGGGGCC TGACAACAAG CCTCTTGCAG ATAACCGGGA
   1151 TAAAAACGGG AAACTTCAGA AACCTCTTGC CGCCACGCAG ATGGGACTTA
   1201 TTTATGTCAA TCCTGAAGGC CCCGGTGGAA AACCAGATCC TCTGGCTTCC
   1251 GCGAAAGATA TCAGGGAAGC TTTTTCACGT ATGGCCATGG ATGATGAGGA
   1301 GACTGTGGCC CTGATCGCGG GAGGGCATAC ATTTGGTAAA GCACATGGTG
   1351 CAGCGTCTCC TGAAAAATGT ATTGGCGCAG GGCCTGATGG TGCACCTGTG
   1401 GAGGAGCAGG GACTGGGATG GAAAAATAAA TGTGGTACAG GAAACGGCAA
   1451 ATATACCATC ACCAGTGGCC TGGAAGGAGC CTGGTCGAC
(2) INFORMATION POUR LA SEQUENCE ID N°2 :
   (i) CARACTERISTIQUE DE LA SEQUENCE :
      (A) LONGUEUR : 1181 paires de bases
      (B) TYPE : acide nucléique
      (C) NOMBRE DE BRINS : double
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°2 : (2) INFORMATION POUR LA SEQUENCE ID N°3 :
      (i) CARACTERISTIQUE DE LA SEQUENCE :
         (A) LONGUEUR : 22 paires de bases
         (B) TYPE : acide nucléique
         (C) NOMBRE DE BRINS : simple
         (D) CONFIGURATION : linéaire
         (E) BRIN : sens
      (ii) TYPE DE MOLECULE : ADN (génomique)
      (iii) DESCRIPTION DE LA SEQUENCE : SEQ ID N°3 : CGGAGATGAAAGCACCACTGTG
(2) INFORMATION POUR LA SEQUENCE ID N°4 :
   (i) CARACTERISTIQUE DE LA SEQUENCE :
      (A) LONGUEUR : 22 paires de bases
      (B) TYPE : acide nucléique
      (C) NOMBRE DE BRINS : simple
      (D) CONFIGURATION : linéaire
      (E) BRIN : antisens
   (ii) TYPE DE MOLECULE : ADN (génomique)
   (iii) DESCRIPTION DE LA SEQUENCE : SEQ ID N°4 : GGGCTGTGTAATCTCAGAGGAG
(2) INFORMATION POUR LA SEQUENCE ID N°5 :
   (i) CARACTERISTIQUE DE LA SEQUENCE :
      (A) LONGUEUR : 25 paires de bases
      (B) TYPE : acide nucléique
      (C) NOMBRE DE BRINS : simple
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°5 : GTCCGGAGATGAAAGCACCACTGTG
(2) INFORMATION POUR LA SEQUENCE ID N°6 :
   (i) CARACTERISTIQUE DE LA SEQUENCE :
      (A) LONGUEUR : 25 paires de bases
      (B) TYPE : acide nucléique
      (C) NOMBRE DE BRINS : simple
      (D) CONFIGURATION : linéaire
      (E) BRIN : antisens
   (ii) TYPE DE MOLECULE : ADN (génomique)
   (iii) DESCRIPTION DE LA SEQUENCE : SEQ ID N°6 : TCAGGGCTGTGTAATCTCAGAGGAG
(2) INFORMATION POUR LA SEQUENCE ID N°7 :
   (i) CARACTERISTIQUE DE LA SEQUENCE :
      (A) LONGUEUR : 23 paires de bases
      (B) TYPE : acide nucléique
      (C) NOMBRE DE BRINS : simple
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°7 : GGCGCTGATACCGGCAAGAATGG
(2) INFORMATION POUR LA SEQUENCE ID N°8 :
   (i) CARACTERISTIQUE DE LA SEQUENCE :
      (A) LONGUEUR : 23 paires de bases
      (B) TYPE : acide nucléique
      (C) NOMBRE DE BRINS : simple
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°8 : GGTCCCGCAGGCCATGATTTTTG
(2) INFORMATION POUR LA SEQUENCE ID N°9 :
   (i) CARACTERISTIQUE DE LA SEQUENCE :
      (A) LONGUEUR : 24 paires de bases
      (B) TYPE : acide nucléique
      (C) NOMBRE DE BRINS : simple
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°9 : CCGGCAAGAATGGTCGCAAACTCC
(2) INFORMATION POUR LA SEQUENCE ID N°10 :
   (i) CARACTERISTIQUE DE LA SEQUENCE :
      (A) LONGUEUR : 26 paires de bases
      (B) TYPE : acide nucléique
      (C) NOMBRE DE BRINS: simple
      (D) CONFIGURATION : linéaire
      (E) BRIN : antisens
   (ii) TYPE DE MOLECULE : ADN (génomique)
   (iii) DESCRIPTION DE LA SEQUENCE : SEQ ID N°10 : AAGGGGTTCCAAGCCGCAACTGACGA
(2) INFORMATION POUR LA SEQUENCE ID N°11 :
   (i) CARACTERISTIQUE DE LA SEQUENCE :
      (A) LONGUEUR : 26 paires de bases
      (B) TYPE : acide nucléique
      (C) NOMBRE DE BRINS : simple
      (D) CONFIGURATION : linéaire
      (E) BRIN : antisens
   (ii) TYPE DE MOLECULE : ADN (génomique)
   (iii) DESCRIPTION DE LA SEQUENCE : SEQ ID N°11 : TAAGGGGTTCCAAGCCGCAACTGACG
(2) INFORMATION POUR LA SEQUENCE ID N°12 :
   (i) CARACTERISTIQUE DE LA SEQUENCE :
      (A) LONGUEUR : 31 paires de bases
      (B) TYPE : acide nucléique
      (C) NOMBRE DE BRINS : simple
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°12 : CTCAACGGCATCGTCAGTTGCGGCTTGGAAC
(2) INFORMATION POUR LA SEQUENCE ID N°13 :
   (i) CARACTERISTIQUE DE LA SEQUENCE :
      (A) LONGUEUR : 31 paires de bases
      (B) TYPE : acide nucléique
      (C) NOMBRE DE BRINS : simple
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°13 : AGCACTCAACGGCATCGTCAGTTGCGGCTTG
(2) INFORMATION POUR LA SEQUENCE ID N°14 :
   (i) CARACTERISTIQUE DE LA SEQUENCE :
      (A) LONGUEUR : 31 paires de bases
      (B) TYPE : acide nucléique
      (C) NOMBRE DE BRINS : simple
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°14 : CTATTTCAGGATACCCTTCGTCATCAACACG
(2) INFORMATION POUR LA SEQUENCE ID N°15 :
   (i) CARACTERISTIQUE DE LA SEQUENCE :
      (A) LONGUEUR : 31 paires de bases
      (B) TYPE : acide nucléique
      (C) NOMBRE DE BRINS : simple
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°15 : AATTTCCCTTAATCCGGAGCTATTCGTATGA
(2) INFORMATION POUR LA SEQUENCE ID N°16 :
   (i) CARACTERISTIQUE DE LA SEQUENCE :
      (A) LONGUEUR : 20 paires de bases
      (B) TYPE : acide nucléique
      (C) NOMBRE DE BRINS : simple
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°16 : GAAGACCAGCTTTTTGTTTC
(2) INFORMATION POUR LA SEQUENCE ID N°17 :
   (i) CARACTERISTIQUE DE LA SEQUENCE :
      (A) LONGUEUR : 20 paires de bases
      (B) TYPE : acide nucléique
      (C) NOMBRE DE BRINS : simple
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°17 : TGTCACAGACTCAATGACTA
(2) INFORMATION POUR LA SEQUENCE ID N°18 :
   (i) CARACTERISTIQUE DE LA SEQUENCE :
      (A) LONGUEUR : 14 paires de bases
      (B) TYPE : acide nucléique
      (C) NOMBRE DE BRINS : simple
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°18 : GGCATCGTCAGTTG
(2) INFORMATION POUR LA SEQUENCE ID N°19 :
   (i) CARACTERISTIQUE DE LA SEQUENCE :
      (A) LONGUEUR : 16 paires de bases
      (B) TYPE : acide nucléique
      (C) NOMBRE DE BRINS : simple
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°19 : CGGCATCGTCAGTTGC
(2) INFORMATION POUR LA SEQUENCE ID N°20 :
   (i) CARACTERISTIQUE DE LA SEQUENCE :
      (A) LONGUEUR : 18 paires de bases
      (B) TYPE : acide nucléique
      (C) NOMBRE DE BRINS : simple
      (D) CONFIGURATION : linéaire
      (E) BRIN: sens
   (ii) TYPE DE MOLECULE : ADN (génomique)
   (iii) DESCRIPTION DE LA SEQUENCE : SEQ ID N°20 : ACGGCATCGTCAGTTGCG
(2) INFORMATION POUR LA SEQUENCE ID N°21 :
   (i) CARACTERISTIQUE DE LA SEQUENCE :
      (A) LONGUEUR : 22 paires de bases
      (B) TYPE : acide nucléique
      (C) NOMBRE DE BRINS : simple
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°21 : CCACCTGAACGATAAGCGGAAC
(2) INFORMATION POUR LA SEQUENCE ID N°22 :
   (i) CARACTERISTIQUE DE LA SEQUENCE :
      (A) LONGUEUR : 22 paires de bases
      (B) TYPE : acide nucléique
      (C) NOMBRE DE BRINS : simple
      (D) CONFIGURATION : linéaire
      (E) BRIN : antisens
   (ii) TYPE DE MOLECULE : ADN (génomique)
   (iii) DESCRIPTION DE LA SEQUENCE : SEQ ID N°22 : CACCTTCCTTCCATCCTCAGAC
(2) INFORMATION POUR LA SEQUENCE ID N°23 :
   (i) CARACTERISTIQUE DE LA SEQUENCE :
      (A) LONGUEUR : 20 paires de bases
      (B) TYPE : acide nucléique
      (C) NOMBRE DE BRINS : simple
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°23 : ATCCCAGCGCGCTCCAGCTG
(2) INFORMATION POUR LA SEQUENCE ID N°24 :
   (i) CARACTERISTIQUE DE LA SEQUENCE :
      (A) LONGUEUR : 22 paires de bases
      (B) TYPE : acide nucléique
      (C) NOMBRE DE BRINS : simple
      (D) CONFIGURATION : linéaire
      (E) BRIN : antisens
   (ii) TYPE DE MOLECULE : ADN (génomique)
   (iii) DESCRIPTION DE LA SEQUENCE : SEQ ID N°24 : ACCCATGATGGCGCATCTGATG
(2) INFORMATION POUR LA SEQUENCE ID N°25 :
   (i) CARACTERISTIQUE DE LA SEQUENCE :
      (A) LONGUEUR : 31 paires de bases
      (B) TYPE : acide nucléique
      (C) NOMBRE DE BRINS : simple
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°25 : ACGTTCTGGTCTTACGGGTGATGTAGGTTTT
(2) INFORMATION POUR LA SEQUENCE ID N°26 :
   (i) CARACTERISTIQUE DE LA SEQUENCE :
      (A) LONGUEUR : 31 paires de bases
      (B) TYPE : acide nucléique
      (C) NOMBRE DE BRINS : simple
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°26 : TAGTGAAGCGGTGACAGCATATCAGACGGCT
(2) INFORMATION POUR LA SEQUENCE ID N°27 :
   (i) CARACTERISTIQUE DE LA SEQUENCE :
      (A) LONGUEUR : 21 paires de bases
      (B) TYPE : acide nucléique
      (C) NOMBRE DE BRINS : simple
      (D) CONFIGURATION : linéaire
      (E) BRIN : sens
   (ii) TYPE DE MOLECULE : ADN (génomique)
   (iii) DESCRIPTION DE LA SEQUENCE : SEQ ID N°27 : GTGAGATAGGCACAACAATGA

## Revendications

1. Acide nucléique isolé consistant en la séquence nucléotidique SEQ ID N°1 ou la séquence nucléotidique SEQ ID N°2, leurs séquences complémentaires, et les fragments d'au moins 8 nucléotides consécutifs de SEQ ID N°2 et les séquences dérivées de SEQ ID N°2, différant par mutation, insertion, délétion et/ou substitution d'une ou plusieurs bases et s'hybridant dans des conditions de forte stringence avec la séquence SEQ ID N°2.

2. Acide nucléique isolé consistant en un fragment d'au moins 14 nucléotides consécutifs de la séquence nucléotidique SEQ ID N°1, ou une séquence dérivée de la séquence nucléotidique SEQ ID N°1, différant par délétion et/ou substitution d'une ou plusieurs bases et s'hybridant dans des conditions de forte stringence avec la séquence SEQ ID N°1,
ledit fragment ou ladite séquence dérivée comprenant un enchaînement nucléotidique résultant de l'association stable d'au moins une partie de la séquence d'insertion IS91 et au moins une partie de la séquence du gène katP.

3. Acide nucléique isolé selon la revendication 1 ou 2, comprenant au moins 14 nucléotides consécutifs de l'enchaînement de la séquence SEQ ID N° 1, incluant les nucléotides de la position 400 à 407.

4. Acide nucléique isolé comprenant au moins 8 nucléotides consécutifs de la séquence SEQ ID N°2, ou de la séquence complémentaire et de séquences dérivées de SEQ ID N°2, telles que définies à la revendication 1.

5. Acide nucléique selon la revendication 4 comprenant au moins 14 nucléotides consécutifs de la séquence SEQ ID NO: 2.

6. Acide nucléique isolé selon l'une des revendications 3 à 5, choisi parmi les séquences nucléotidiques suivantes :
SEQ ID N°10 :5'-AAGGGGTTCCAAGCCGCAACTGACGA-3'
SEQ ID N°11 : 5'-TAAGGGGTTCCAAGCCGCAACTGACG-3'
SEQ ID N°12 :5'-CTCAACGGCATCGTCAGTTGCGGCTTGGAAC-3'
SEQ ID N° 1 3 : 5'-AGCACTCAACGGCATCGTCAGTTGCGGCTTG-3'
SEQ ID N°18 : 5'-GGCATCGTCAGTTG-3'
SEQ ID N°19 : 5'-CGGCATCGTCAGTTGC-3'
SEQ ID N°20 : 5'-ACGGCATCGTCAGTTGCG-3'
SEQ ID N°21 : 5'-CCACCTGAACGATAAGCGGAAC-3'
SEQ ID N°22 : 5'-CACCTTCCTTCCATCCTCAGAC-3'
SEQ ID N°23 : 5'-ATCCCAGCGCGCTCCAGCTG-3'
SEQ ID N°24 : 5'-ACCCATGATGGCGCATCTGATG-3'
SEQ ID N25 : 5'-ACGTTCTGGTCTTACGGGTGATGTAGGTTTT-3'
SEQ ID N°26 : 5'-TAGTGAAGCGGTGACAGCATATCAGACGGCT-3'
SEQ ID N° 27:5'-GTGAGATAGGCACAACAATGA-3'.

7. Couples d'acides nucléiques isolés, utilisés comme amorces, choisis parmi des couples suivants de séquences suivantes :
- SEQ ID N°3 et SEQ ID N°4
- SEQ ID N°5 et SEQ ID N°6
- SEQ ID N°6 et SEQ ID N°7
- SEQ ID N°6 et SEQ ID N°8
- SEQ ID N°6 et SEQ ID N°9
- SEQ ID N°21 et SEQ ID N°22
- SEQ ID N°23 et SEQ ID N°24.

8. Plasmides pDF3 et pDF4 déposés à la Collection Nationale de Cultures de Microorganismes respectivement sous les numéros 1-1999 et 1-2000, le 26 mars 1998.

9. Cellule hôte comprenant un plasmide selon la revendication 8.

10. Procédé de détection d'une E. Coli entérohémorragique (EHEC) dans un échantillon, ladite méthode comprenant la détection d'une chaîne nucléotidique de séquence SEQ ID N°2 avec un acide nucléique isolé tel que défini dans la revendication 4, la présence de ladite chaîne nucléotidique étant révélatrice de la présence d'une EHEC.

11. Procédé selon la revendication 10, dans lequel la séquence dudit acide nucléique isolé est sélectionnée dans le groupe consistant en :
SEQ ID N°21 : 5'-CCACCTGAACGATAAGCGGAAC-3'
SEQ ID N°22 : 5'-CACCTTCCTTCCATCCTCAGAC-3'
SEQ ID N°23 : 5'-ATCCCAGCGCGCTCCAGCTG-3'
SEQ ID N°24 : 5'-ACCCATGATGGCGCATCTGATG-3'
SEQ ID N°25 : 5'-ACGTTCTGGTCTTACGGGTGATGTAGGTTTT-3'
SEQ ID N°26 : 5'-TAGTGAAGCGGTGACAGCATATCAGACGGCT-3' ; et
SEQ ID N° 27 : 5'-GTGAGATAGGCACAACAATGA-3'.

12. Procédé selon la revendication 10 ou 11, dans lequel ledit acide nucléique isolé est utilisé en tant qu'amorce et/ou sonde.

13. Procédé selon l'une quelconque des revendications 10 à 12, dans lequel ledit acide nucléique isolé est marqué.

14. Procédé selon l'une quelconque des revendications 10 à 13, dans lequel ledit acide nucléique isolé est immobilisé sur un support.

15. Procédé de détection des EHEC dans un échantillon, comprenant les étapes suivantes :
(a) mise en contact de l'échantillon avec un couple d'amorces oligonucléotiques sélectionnés dans le groupe consistant en :
SEQ ID N°21 : 5'-CCACCTGAACGATAAGCGGAAC-3'
SEQ ID N°22 : 5'-CACCTTCCTTCCATCCTCAGAC-3'
SEQ ID N°23 : 5'-ATCCCAGCGCGCTCCAGCTG-3'
SEQ ID N°24 : 5'-ACCCATGATGGCGCATCTGATG-3'
SEQ ID N°25 : 5'-ACGTTCTGGTCTTACGGGTGATGTAGGTTTT-3' SEQ ID N°26 : 5'-TAGTGAAGCGGTGACAGCATATCAGACGGCT-3' ; et
SEQ ID N°27 : 5'-GTGAGATAGGCACAACAATGA-3'
l'acide nucléique contenu dans l'échantillon ayant été, le cas échéant, rendu accessible aux dites amorces à la cible recherchée,
(b) amplification de la séquence nucléotidique encadrée par le couple d'amorces choisi,
(c) vérification de la présence du produit amplifié par utilisation d'au moins une sonde spécifique du produit amplifié.

16. Procédé selon la revendication 15, selon lequel l'étape (c) comprend les sous-étapes suivantes :
(c1) dénaturation des séquences amplifiées par un moyen physique ou chimique ;
(c2) mise en contact avec une solution contenant les fragments amplifiés dénaturés de l'étape (c1) avec, d'une part, au moins une sonde de capture, et d'autre part, au moins une sonde de détection, éventuellement marquée, les sondes de capture et de détection étant susceptibles de s'hybrider avec le même brin des fragments amplifiés, ladite mise en contact étant réalisée pendant un temps suffisant pour permettre la réaction d'hybridation ;
(c3) au moins un lavage pour éliminer les séquences nucléotidiques n'ayant pas réagi ;
(c4) révélation des sondes de détection hybridées aux séquences nucléotidiques amplifiées.

17. Procédé selon la revendication 16, dans lequel la sonde de capture est fixée à la surface d'un puits d'une plaque de microtitration.

18. Procédé selon la revendication 16 ou 17, dans lequel la sonde de détection est marquée à la péroxydase.

19. Procédé selon la revendication 18, **caractérisé en ce que** la mise en évidence de l'activité de la péroxydase liée à la sonde de détection ayant réagi, s'effectue par réaction colorimétrique, en présence d'un substrat chromogène, tel que le tétraméthylbenzidine (TMB), par la mise en oeuvre des étapes suivantes :
- addition du substrat chromogène, tel qu'une solution de TMB dans les puits contenant le mélange réactionnel,
- incubation, à l'obscurité, pendant un temps suffisant pour permettre le développement de la coloration,
- blocage de la réaction par addition d'une solution d'arrêt,
- détermination de la densité optique à une longueur d'onde appropriée.

20. Procédé de détection des EHEC, selon l'une quelconque des revendications 16 à 19, mettant en oeuvre les oligonucléotides suivants :
- les séquences SEQ ID N°21 et SEQ ID N°22, comme amorces pour l'amplification,
- la séquence SEQ ID N°25, comme sonde de capture, et
- la séquence SEQ ID N°27, comme sonde de détection.

21. Procédé de détection de *E. coli* 0157:H7 dans un échantillon, ledit procédé comprenant la détection d'une chaîne nucléotidique résultant de l'association stable d'au moins une partie de la séquence d'insertion IS91 et au moins une partie de la séquence du gène *katP* à l'aide d'un acide nucléique isolé comprenant au moins 8 nucléotides consécutifs de la séquence SEQ ID N°1, de la séquence complémentaire de celle-ci ou d'une séquence dérivée de celle-ci, différant par délétion et/ou substitution d'une ou plusieurs bases et s'hybridant dans des conditions de forte stringence avec la séquence SEQ ID N°1, la présence de ladite chaîne nucléotidique étant révélatrice de la présence de *E. coli* 0157 :H7.

22. Procédé selon la revendication 21, dans lequel la séquence dudit acide nucléique isolé est sélectionnée dans le groupe consistant en :
SEQ ID N°3 : 5'-CGGAGATGAAAGCACCACTGTG-3'
SEQ ID N°4 : 5'-GGGCTGTGTAATCTCAGAGGAG-3'
SEQ ID N°5 : 5'-GTCCGGAGATGAAAGCACCACTGTG-3'
SEQ ID N°6 : 5'-TCAGGGCTGTGTAATCTCAGAGGAG-3'
SEQ ID N°7 : 5'-GGCGCTGATACCGGCAAGAATGG-3'
SEQ ID N°8 : 5'-GGTCCCGCAGGCCATGATTTTTG-3'
SEQ ID N°9 : 5'-CCGGCAAGAATGGTCGCAAACTCC-3'
SEQ ID N°10 :5'-AAGGGGTTCCAAGCCGCAACTGACGA-3'
SEQ ID N°11 : 5'-TAAGGGGTTCCAAGCCGCAACTGACG-3'
SEQ ID N°12 :5'-CTCAACGGCATCGTCAGTTGCGGCTTGGAAC-3'
SEQ ID N°13 : 5'-AGCACTCAACGGCATCGTCAGTTGCGGCTTG-3'
SEQ ID N°14 :5'-CTATTTCAGGATACCCTTCGTCATCAACACG-3'
SEQ ID N°15:5'-AATTTCCCTTAATCCGGAGCTATTCGTATGA-3'SEQID
SEQ ID N°16 : 5'-GAAGACCAGCTTTTTGTTTC-3'
S EQ ID N°17 : 5'-TGTCACAGACTCAATGACTA-3'
SEQ ID N°18 : 5'-GGCATCGTCAGTTG-3'
SEQ ID N°19 : 5'-CGGCATCGTCAGTTGC-3' ; et
SEQ ID N°20 : 5'-ACGGCATCGTCAGTTGCG-3.

23. Procédé selon la revendication 21 ou 22, dans lequel ledit acide nucléique isolé est utilisé comme amorce et/ou sonde.

24. Procédé selon l'une quelconque des revendications 21 à 23, dans lequel ledit acide nucléique isolé est marqué.

25. Procédé selon l'une quelconque des revendications 21 à 23, dans lequel ledit acide nucléique isolé est immobilisé sur un support.

26. Procédé selon la revendication 21, comprenant les étapes suivantes :
(a) mettre en contact de l'échantillon avec une paire d'amorces oligonucléotidiques sélectionnée dans le groupe consistant en :
SEQ ID N°3 : 5'-CGGAGATGAAAGCACCACTGTG-3'
SEQ ID N°4 : 5'-GGGCTGTGTAATCTCAGAGGAG-3'
SEQ ID N°5 : 5'-GTCCGGAGATGAAAGCACCACTGTG-3'
SEQ ID N°6 : 5'-TCAGGGCTGTGTAATCTCAGAGGAG-3'
SEQ ID N°7 : 5'-GGCGCTGATACCGGCAAGAATGG-3'
SEQ ID N°8 : 5'-GGTCCCGCAGGCCATGATTTTTG-3'
SEQ ID N°9 : 5'-CCGGCAAGAATGGTCGCAAACTCC-3'
SEQ ID N°10 :5'-AAGGGGTTCCAAGCCGCAACTGACGA-3'
SEQ ID N°11 : 5'-TAAGGGGTTCCAAGCCGCAACTGACG-3'
SEQ ID N°12 :5'-CTCAACGGCATCGTCAGTTGCGGCTTGGAAC-3'
SEQ ID N°13 : 5'-AGCACTCAACGGCATCGTCAGTTGCGGCTTG-3'
SEQ ID N°18 : 5'-GGCATCGTCAGTTG-3'
SEQ ID N°19 : 5'-CGGCATCGTCAGTTGC-3' ; et
SEQ ID N°20 : 5'-ACGGCATCGTCAGTTGCG-3 ;
l'acide nucléique contenu dans l'échantillon ayant été, le cas échéant, rendu accessible auxdites amorces à la cible recherchée ;
b) amplifier la séquence nucléotidique encadrée par le couple d'amorces choisi ;
c) vérifier la présence du produit amplifié par utilisation d'au moins une sonde spécifique du produit amplifié.

27. Procédé selon la revendication 26, selon lequel l'étape (c) comprend les sous-étapes suivantes :
(c1) dénaturation des séquences amplifiées par un moyen physique ou chimique ;
(c2) mise en contact avec une solution contenant les fragments amplifiés dénaturés de l'étape (c1) avec, d'une part, au moins une sonde de capture, et d'autre part, au moins une sonde de détection, éventuellement marquée, les sondes de capture et de détection étant susceptibles de s'hybrider avec le même brin des fragments amplifiés, ladite mise en contact étant réalisée pendant un temps suffisant pour permettre la réaction d'hybridation ;
(c3) au moins un lavage pour éliminer les séquences nucléotidiques n'ayant pas réagi ;
(c4) révélation des sondes de détection hybridées aux séquences nucléotidiques amplifiées.

28. Procédé selon la revendication 27, dans lequel la sonde de capture est fixée à la surface d'un puits d'une plaque de microtitration.

29. Procédé selon la revendication 27 ou 28, dans lequel la sonde de détection est marquée à la péroxydase.

30. Procédé selon la revendication 29, **caractérisé en ce que** la mise en évidence de l'activité de la péroxydase liée à la sonde de détection ayant réagi, s'effectue par réaction colorimétrique, en présence d'un substrat chromogène, tel que le tétraméthylbenzidine (TMB), par la mise en oeuvre des étapes suivantes :
- addition du substrat chromogène, tel qu'une solution de TMB dans les puits contenant le mélange réactionnel,
- incubation, à l'obscurité, pendant un temps suffisant pour permettre le développement de la coloration,
- blocage de la réaction par addition d'une solution d'arrêt,
- détermination de la densité optique à une longueur d'onde appropriée.

31. Procédé de détection de E. coli O157 : H7, selon l'une quelconque des revendications 27 à 30, mettant en oeuvre les oligonudéotides suivants :
- les séquences. SEQ ID N°5 et SEQ ID N°6, comme amorces pour l'amplification,
- la séquence SEQ ID N°15, comme sonde de capture,
- la séquence SEQ ID N°18, comme sonde de détection.

32. Trousse pour la détection des EHECs, comprenant parmi les réactifs :
- au moins deux amorces nucléotidiques sélectionnées dans le groupe consistant en :
SEQ ID N°21 : 5'-CCACCTGAACGATAAGCGGAAC-3'
SEQ ID N°22 : 5'-CACCTTCCTTCCATCCTCAGAC-3'
SEQ ID N°23 : 5'-ATCCCAGCGCGCTCCAGCTG-3'
SEQ ID N°24 : 5'-ACCCATGATGGCGCATCTGATG-3'
SEQ ID N°25 : 5'-ACGTTCTGGTCTTACGGGTGATGTAGGTTTT-3'
SEQ ID N°26 : 5'-TAGTGAAGCGGTGACAGCATATCAGACGGCT-3'
SEQ ID N°27 : 5'-GTGAGATAGGCACAACAATGA-3'; et
- éventuellement au moins une sonde nucléotidique sélectionnée dans le groupe consistant en :
SEQ ID N°21 : 5'-CCACCTGAACGATAAGCGGAAC-3'
SEQ ID N°22 : 5'-CACCTTCCTTCCATCCTCAGAC-3'
SEQ ID N°23 : 5'-ATCCCAGCGCGCTCCAGCTG-3'
SEQ ID N°24 : 5'-ACCCATGATGGCGCATCTGATG-3'
SEQ ID N°25 : 5'-ACGTTCTGGTCTTACGGGTGATGTAGGTTTT-3'
SEQ ID N°26 : 5'-TAGTGAAGCGGTGACAGCATATCAGACGGCT-3'
SEQ ID N°27 : 5'-GTGAGATAGGCACAACAATGA-3' ;
pour la détection du produit amplifié.

33. Trousse pour la détection des EHECs, selon la revendication 32 comprenant :
- deux oligonucléotides de séquences SEQ ID N°21 et SEQ ID N° 22, utilisés comme paire d'amorces,
- deux oligonucléotides de séquences SEQ ID N° 25 et SEQ ID N° 27, pour la détection du produit amplifié.

34. Trousse pour la détection de *E. coli* O157:H7, comprenant parmi les réactifs:
- au moins deux amorces oligonucléotidiques sélectionnées dans le groupe consistant en :
SEQ ID N°3 : 5'-CGGAGATGAAAGCACCACTGTG-3'
SEQ ID N°4 : 5'-GGGCTGTGTAATCTCAGAGGAG-3'
SEQ ID N°5 : 5'-GTCCGGAGATGAAAGCACCACTGTG-3'
SEQ ID N°6 : 5'-TCAGGGCTGTGTAATCTCAGAGGAG-3'
SEQ ID N°7 : 5'-GGCGCTGATACCGGCAAGAATGG-3'
SEQ ID N°8 : 5'-GGTCCCGCAGGCCATGATTTTTG-3'
SEQ ID N°9 : 5'-CCGGCAAGAATGGTCGCAAACTCC-3'
SEQ ID N°10 :5'-AAGGGGTTCCAAGCCGCAACTGACGA-3'
SEQ ID N°11 : 5'-TAAGGGGTTCCAAGCCGCAACTGACG-3'
SEQ ID N°12 :5'-CTCAACGGCATCGTCAGTTGCGGCTTGGAAC-3'
SEQ ID N°13 : 5'-AGCACTCAACGGCATCGTCAGTTGCGGCTTG-3'
SEQ ID N°14 :5'-CTATTTCAGGATACCCTTCGTCATCAACACG-3'
SEQ ID N°15:5'-AATTTCCCTTAATCCGGAGCTATTCGTATGA-3'SEQID
SEQ ID N°16 : 5'-GAAGACCAGCTTTTTGTTTC-3'
SEQ ID N°17 : 5'-TGTCACAGACTCAATGACTA-3'
SEQ ID N°18 : 5'-GGCATCGTCAGTTG-3'
SEQ ID N°19 : 5'-CGGCATCGTCAGTTGC-3' ; et
SEQ ID N°20 : 5'-ACGGCATCGTCAGTTGCG-3 ;
et
- éventuellement au moins une amorce oligonucléotidique sélectionnée dans le groupe consistant en :
SEQ ID N°3 : 5'-CGGAGATGAAAGCACCACTGTG-3'
SEQ ID N°4 5'-GGGCTGTGTAATCTCAGAGGAG-3'
SEQ ID N°5 : 5'-GTCCGGAGATGAAAGCACCACTGTG-3' SEQ ID N°6 : 5'-TCAGGGCTGTGTAATCTCAGAGGAG-3' SEQ ID N°7 : 5'-GGCGCTGATACCGGCAAGAATGG-3'
SEQ ID N°8 : 5'-GGTCCCGCAGGCCATGATTTTTG-3'
SEQ ID N°9 : 5'-CCGGCAAGAATGGTCGCAAACTCC-3'
SEQ ID N°10 :5'-AAGGGGTTCCAAGCCGCAACTGACGA-3'
SEQ ID N°11 : 5'-TAAGGGGTTCCAAGCCGCAACTGACG-3'
SEQ ID N°12 :5'-CTCAACGGCATCGTCAGTTGCGGCTTGGAAC-3'
SEQ ID N°13 : 5'-AGCACTCAACGGCATCGTCAGTTGCGGCTTG-3'
SEQ ID N°14 :5'-CTATTTCAGGATACCCTTCGTCATCAACACG-3'
SEQ ID N°15:5'-AATTTCCCTTAATCCGGAGCTATTCGTATGA-3'SEQID
SEQ ID N°16 : 5'-GAAGACCAGCTTTTTGTTTC-3'
SEQ ID N°17 : 5'-TGTCACAGACTCAATGACTA-3'
SEQ ID N°18 : 5'-GGCATCGTCAGTTG-3'
SEQ ID N°19 : 5'-CGGCATCGTCAGTTGC-3'
SEQ ID N°20 : 5'-ACGGCATCGTCAGTTGCG-3 ; et
pour la détection du produit amplifié.

35. Trousse pour la détection de *E. coli* O157:H7 selon la revendication 34 comprenant :
- deux oligonucléotides de séquences SEQ ID N°5 et SEQ ID N°6, utilisés comme paire d'amorces ; et
- deux oligonucléotides de séquences SEQ ID N°15 et SEQ ID N°18 pour la détection du produit amplifié.

## Claims

1. Isolated nucleic acid consisting of nucleotide sequence SEQ ID No. 1 or nucleotide sequence SEQ ID No. 2, the sequences complimentary thereto, and the fragments of at least 8 consecutive nucleotides of SEQ ID No. 2 and sequences derived from SEQ ID No. 2 differing by mutation, insertion, deletion and/or substitution of one or more bases and hybridising with sequence SEQ ID No. 2 under high stringency conditions.

2. Isolated nucleic acid consisting of a fragment of at least 14 consecutive nucleotides of nucleotide sequence SEQ ID No. 1 or a sequence derived from nucleotide sequence SEQ ID No. 1 differing by deletion and/or substitution of one or more bases and hybridising with sequence SEQ ID No. 1 under high stringency conditions, said fragment or said derived sequence comprising a nucleotide chain resulting from the stable combination of at least a portion of the insertion sequence IS91 and at least a portion of the gene sequence *katP.*

3. Isolated nucleic acid according to claim 1 or 2, comprising at least 14 consecutive nucleotides of the chain of sequence SEQ ID No. 1, including the nucleotides from position 400 to 407.

4. Isolated nucleic acid comprising at least 8 consecutive nucleotides of sequence SEQ ID No. 2, or of the sequence complementary thereto and of sequences derived from SEQ ID No. 2, as defined in claim 1.

5. Nucleic acid according to claim 4 comprising at least 14 consecutive nucleotides of sequence SEQ ID No. 2.

6. Isolated nucleic acid according to any one of claims 3 to 5, selected from the following nucleotide sequences:
SEQ ID No. 10: 5'-AAGGGGTTCCAAGCCGCAACTGACGA-3'
SEQ ID No. 11: 5'-TAAGGGGTTCCAAGCCGCAACTGACG-3'
SEQ ID No. 12: 5'-CTCAACGGCATCGTCAGTTGCGGCTTGGAAC-3'
SEQ ID No. 13: 5'-AGCACTCAACGGCATCGTCAGTTGCGGCTTG-3'
SEQ ID No. 18: 5'-GGCATCGTCAGTTG-3'
SEQ ID No. 19: 5'-CGGCATCGTCAGTTGC-3'
SEQ ID No. 20: 5'-ACGGCATCGTCAGTTGCG-3'
SEQ ID No. 21: 5'-CCACCTGAACGATAAGCGGAAC-3'
SEQ ID No. 22: 5'-CACCTTCCTTCCATCCTCAGAC-3'
SEQ ID No. 23: 5'-ATCCCAGCGCGCTCCAGCTG-3'
SEQ ID No. 24: 5'-ACCCATGATGGCGCATCTGATG-3'
SEQ ID No. 25: 5'-ACGTTCTGGTCTTACGGGTGATGTAGGTTTT-3'
SEQ ID No. 26: 5'-TAGTGAAGCGGTGACAGCATATCAGACGGCT-3'
SEQ ID No. 27: 5'-GTGAGATAGGCACAACAATGA-3'.

7. Pairs of isolated nucleic acids, used as primers, selected from the following pairs of sequences:
- SEQ ID No. 3 and SEQ ID No. 4
- SEQ ID No. 5 and SEQ ID No. 6
- SEQ ID No. 6 and SEQ ID No. 7
- SEQ ID No. 6 and SEQ ID No. 8
- SEQ ID No. 6 and SEQ ID No. 9
- SEQ ID No. 21 and SEQ ID No. 22
- SEQ ID No. 23 and SEQ ID No. 24.

8. Plasmids pDF3 and pDF4 deposited on 26 March 1998 at the Collection Nationale de Cultures de Microorganismes under numbers 1-1999 and 1-2000 respectively.

9. Host cell comprising a plasmid according to claim 8.

10. Method of detecting an enterohaemorrhagic *E. coli* (EHEC) in a sample, said method comprising the detection of a nucleotide chain of sequence SEQ ID No. 2 with an isolated nucleic acid as defined in claim 4, the presence of said nucleotide chain indicating the presence of an EHEC.

11. Method according to claim 10, wherein the sequence of said isolated nucleic acid is selected from the group consisting of:
SEQ ID No. 21: 5'-CCACCTGAACGATAAGCGGAAC-3'
SEQ ID No. 22: 5'-CACCTTCCTTCCATCCTCAGAC-3'
SEQ ID No. 23: 5'-ATCCCAGCGCGCTCCAGCTG-3'
SEQ ID No. 24: 5'-ACCCATGATGGCGCATCTGATG-3'
SEQ ID No. 25: 5'-ACGTTCTGGTCTTACGGGTGATGTAGGTTTT-3' SEQ ID No. 26: 5'-TAGTGAAGCGGTGACAGCATATCAGACGGCT-3'; and
SEQ ID No. 27: 5'-GTGAGATAGGCACAACAATGA-3'.

12. Method according to claim 10 or 11, wherein said isolated nucleic acid is used as primer and/or probe.

13. Method according to any one of claims 10 to 12, wherein said isolated nucleic acid is labelled.

14. Method according to any one of claims 10 to 13, wherein said isolated nucleic acid is immobilised on a support.

15. Method of detecting EHECs in a sample, comprising the following steps:
(a) bringing the sample into contact with a pair of oligonucleotide primers selected from the group consisting of:
SEQ ID No. 21: 5'-CCACCTGAACGATAAGCGGAAC-3'
SEQ ID No. 22: 5'-CACCTTCCTTCCATCCTCAGAC-3'
SEQ ID No. 23: 5'-ATCCCAGCGCGCTCCAGCTG-3'
SEQ ID No. 24: 5'-ACCCATGATGGCGCATCTGATG-3'
SEQ ID No. 25: 5'-ACGTTCTGGTCTTACGGGTGATGTAGGTTTT-3' SEQ ID No. 26: 5'-TAGTGAAGCGGTGACAGCATATCAGACGGCT-3'; and
SEQ ID No. 27: 5'-GTGAGATAGGCACAACAATGA-3',
the nucleic acid contained in the sample having been, where appropriate, made accessible to said primers having the target tested for,
(b) amplifying the nucleotide sequence flanked by the chosen pair of primers,
(c) verifying the presence of the amplified product by using at least one probe specific to the amplified product.

16. Method according to claim 15, according to which step (c) comprises the following subsidiary steps:
(c1) denaturing the amplified sequences by a physical or chemical means;
(c2) contacting a solution containing the denatured amplified fragments of step (c1) with, on the one hand, at least one capture probe and, on the other hand, at least one detection probe, optionally labelled, the capture and detection probes being capable of hybridising with the same strand of the amplified fragments, said contacting being carried out for a period of time sufficient to permit the hybridisation reaction;
(c3) at least one washing in order to remove unreacted nucleotide sequences;
(c4) visualisation of the detection probes hybridised with the amplified nucleotide sequences.

17. Method according to claim 16, wherein the capture probe is fixed to the surface of a well of a microtitre plate.

18. Method according to claim 16 or 17, wherein the detection probe is labelled with peroxidase.

19. Method according to claim 18, **characterised in that** the activity of the peroxidase linked to the detection probe that has reacted is visualised by colorimetric reaction in the presence of a chromogenic substrate, such as tetramethylbenzidine (TMB), by carrying out the following steps:
- addition of the chromogenic substrate, such as a solution of TMB, to the wells containing the reaction mixture,
- incubation, in the dark, for a period of time sufficient to allow the colouration to develop,
- stopping the reaction by addition of a stop solution,
- determination of the optical density at a suitable wavelength.

20. Method of detecting EHECs according to any one of claims 16 to 19, using the following oligonucleotides:
- sequences SEQ ID No. 21 and SEQ ID No. 22 as primers for the amplification,
- sequence SEQ ID No. 25 as capture probe, and
- sequence SEQ ID No. 27 as detection probe.

21. Method of detecting *E. coli* O157:H7 in a sample, said method comprising the detection of a nucleotide chain resulting from the stable combination of at least a portion of the insertion sequence IS91 and at least a portion of the gene sequence *katP* gene using an isolated nucleic acid comprising at least 8 consecutive nucleotides of sequence SEQ ID No. 1, of the sequence complementary thereto or of a sequence derived therefrom differing by deletion and/or substitution of one or more bases and hybridising with sequence SEQ ID No. 1 under high stringency conditions, the presence of said nucleotide chain indicating the presence of *E. coli* O157:H7.

22. Method according to claim 21, wherein the sequence of said isolated nucleic acid is selected from the group consisting of:
SEQ ID No. 3: 5'-CGGAGATGAAAGCACCACTGTG-3'
SEQ ID No. 4: 5'-GGGCTGTGTAATCTCAGAGGAG-3'
SEQ ID No. 5: 5'-GTCCGGAGATGAAAGCACCACTGTG-3'
SEQ ID No. 6: 5'-TCAGGGCTGTGTAATCTCAGAGGAG-3'
SEQ ID No. 7: 5'-GGCGCTGATACCGGCAAGAATGG-3'
SEQ ID No. 8: 5'-GGTCCCGCAGGCCATGATTTTTG-3'
SEQ ID No. 9: 5'-CCGGCAAGAATGGTCGCAAACTCC-3'
SEQ ID No. 10: 5'-AAGGGGTTCCAAGCCGCAACTGACGA-3'
SEQ ID No. 11: 5'-TAAGGGGTTCCAAGCCGCAACTGACG-3'
SEQ ID No. 12: 5'-CTCAACGGCATCGTCAGTTGCGGCTTGGAAC-3'
SEQ ID No. 13: 5'-AGCACTCAACGGCATCGTCAGTTGCGGCTTG-3'
SEQ ID No. 14: 5'-CTATTTCAGGATACCCTTCGTCATCAACACG-3'
SEQ ID No. 15: 5'-AATTTCCCTTAATCCGGAGCTATTCGTATGA-3'SEQID
SEQ ID No. 16: 5'-GAAGACCAGCTTTTTGTTTC-3'
SEQ ID No. 17: 5'-TGTCACAGACTCAATGACTA-3'
SEQ ID No. 18: 5'-GGCATCGTCAGTTG-3'
SEQ ID No. 19: 5'-CGGCATCGTCAGTTGC-3'; and
SEQ ID No. 20: 5'-ACGGCATCGTCAGTTGCG-3'.

23. Method according to claim 21 or 22, wherein said isolated nucleic acid is used as primer and/or probe.

24. Method according to any one of claims 21 to 23, wherein said isolated nucleic acid is labelled.

25. Method according to any one of claims 21 to 23, wherein said isolated nucleic acid is immobilised on a support.

26. Method according to claim 21, comprising the following steps:
(a) bringing the sample into contact with a pair of oligonucleotide primers selected from the group consisting of:
SEQ ID No. 3: 5'-CGGAGATGAAAGCACCACTGTG-3'
SEQ ID No. 4: 5'-GGGCTGTGTAATCTCAGAGGAG-3'
SEQ ID No. 5: 5'-GTCCGGAGATGAAAGCACCACTGTG-3'
SEQ ID No. 6: 5'-TCAGGGCTGTGTAATCTCAGAGGAG-3'
SEQ ID No. 7: 5'-GGCGCTGATACCGGCAAGAATGG-3'
SEQ ID No. 8: 5'-GGTCCCGCAGGCCATGATTTTTG-3'
SEQ ID No. 9: 5'-CCGGCAAGAATGGTCGCAAACTCC-3'
SEQ ID No. 10: 5'-AAGGGGTTCCAAGCCGCAACTGACGA-3'
SEQ ID No. 11: 5'-TAAGGGGTTCCAAGCCGCAACTGACG-3'
SEQ ID No. 12: 5'-CTCAACGGCATCGTCAGTTGCGGCTTGGAAC-3'
SEQ ID No. 13: 5'-AGCACTCAACGGCATCGTCAGTTGCGGCTTG-3'
SEQ ID No. 18: 5'-GGCATCGTCAGTTG-3'
SEQ ID No. 19: 5'-CGGCATCGTCAGTTGC-3'; and
SEQ ID No. 20: 5'-ACGGCATCGTCAGTTGCG-3';
the nucleic acid contained in the sample having been, where appropriate, made accessible to said primers having the target tested for;
b) amplifying the nucleotide sequence flanked by the chosen pair of primers;
c) verifying the presence of the amplified product by using at least one probe specific to the amplified product.

27. Method according to claim 26, according to which step (c) comprises the following subsidiary steps:
(c1) denaturing the amplified sequences by a physical or chemical means;
(c2) contacting a solution containing the denatured amplified fragments of step (c1) with, on the one hand, at least one capture probe and, on the other hand, at least one detection probe, optionally labelled, the capture and detection probes being capable of hybridising with the same strand of the amplified fragments, said contacting being carried out for a period of time sufficient to permit the hybridisation reaction;
(c3) at least one washing in order to remove unreacted nucleotide sequences;
(c4) visualisation of the detection probes hybridised with the amplified nucleotide sequences.

28. Method according to claim 27, wherein the capture probe is fixed to the surface of a well of a microtitre plate.

29. Method according to claim 27 or 28, wherein the detection probe is labelled with peroxidase.

30. Method according to claim 29, **characterised in that** the activity of the peroxidase linked to the detection probe that has reacted is visualised by colorimetric reaction in the presence of a chromogenic substrate, such as tetramethylbenzidine (TMB), by carrying out the following steps:
- addition of the chromogenic substrate, such as a solution of TMB, to the wells containing the reaction mixture,
- incubation, in the dark, for a period of time sufficient to allow the colouration to develop,
- stopping the reaction by addition of a stop solution,
- determination of the optical density at a suitable wavelength.

31. Method of detecting *E. coli* 0157:H7 according to any one of claims 27 to 30, using the following oligonucleotides:
- sequences SEQ ID No. 5 and SEQ ID No. 6 as primers for the amplification,
- sequence SEQ ID No. 15 as capture probe,
- sequence SEQ ID No. 18 as detection probe.

32. Kit for the detection of EHECs, comprising among the reagents:
- at least two nucleotide primers selected from the group consisting of:
SEQ ID No. 21: 5'-CCACCTGAACGATAAGCGGAAC-3'
SEQ ID No. 22: 5'-CACCTTCCTTCCATCCTCAGAC-3'
SEQ ID No. 23: 5'-ATCCCAGCGCGCTCCAGCTG-3'
SEQ ID No. 24: 5'-ACCCATGATGGCGCATCTGATG-3'
SEQ ID No. 25: 5'-ACGTTCTGGTCTTACGGGTGATGTAGGTTTT-3' SEQ ID No. 26: 5'-TAGTGAAGCGGTGACAGCATATCAGACGGCT-3' SEQ ID No. 27: 5'-GTGAGATAGGCACAACAATGA-3'; and
- optionally at least one nucleotide probe selected from the group consisting of:
SEQ ID No. 21: 5'-CCACCTGAACGATAAGCGGAAC-3'
SEQ ID No. 22: 5'-CACCTTCCTTCCATCCTCAGAC-3'
SEQ ID No. 23: 5'-ATCCCAGCGCGCTCCAGCTG-3'
SEQ ID No. 24: 5'-ACCCATGATGGCGCATCTGATG-3'
SEQ ID No. 25: 5'-ACGTTCTGGTCTTACGGGTGATGTAGGTTTT-3'
SEQ ID No. 26: 5'-TAGTGAAGCGGTGACAGCATATCAGACGGCT-3'
SEQ ID No. 27: 5'-GTGAGATAGGCACAACAATGA-3';
for the detection of the amplified product.

33. Kit for the detection of EHECs according to claim 32, comprising:
- two oligonucleotides of sequences SEQ ID No. 21 and SEQ ID No. 22, used as a pair of primers,
- two oligonucleotides of sequences SEQ ID No. 25 and SEQ ID No. 27, for detection of the amplified product.

34. Kit for the detection of *E. coli* 0157:H7, comprising among the reagents:
- at least two oligonucleotide primers selected from the group consisting of:
SEQ ID No. 3: 5'-CGGAGATGAAAGCACCACTGTG-3'
SEQ ID No. 4: 5'-GGGCTGTGTAATCTCAGAGGAG-3'
SEQ ID No. 5: 5'-GTCCGGAGATGAAAGCACCACTGTG-3'
SEQ ID No. 6: 5'-TCAGGGCTGTGTAATCTCAGAGGAG-3'
SEQ ID No. 7: 5'-GGCGCTGATACCGGCAAGAATGG-3'
SEQ ID No. 8: 5'-GGTCCCGCAGGCCATGATTTTTG-3'
SEQ ID No. 9: 5'-CCGGCAAGAATGGTCGCAAACTCC-3'
SEQ ID No. 10: 5'-AAGGGGTTCCAAGCCGCAACTGACGA-3'
SEQ ID No. 11: 5'-TAAGGGGTTCCAAGCCGCAACTGACG-3'
SEQ ID No. 12: 5'-CTCAACGGCATCGTCAGTTGCGGCTTGGAAC-3'
SEQ ID No. 13: 5'-AGCACTCAACGGCATCGTCAGTTGCGGCTTG-3'
SEQ ID No. 14: 5'-CTATTTCAGGATACCCTTCGTCATCAACACG-3'
SEQ ID No. 15: 5'-AATTTCCCTTAATCCGGAGCTATTCGTATGA-3'SEQID
SEQ ID No. 16: 5'-GAAGACCAGCTTTTTGTTTC-3'
SEQ ID No. 17: 5'-TGTCACAGACTCAATGACTA-3'
SEQ ID No. 18: 5'-GGCATCGTCAGTTG-3'
SEQ ID No. 19: 5'-CGGCATCGTCAGTTGC-3'; and
SEQ ID No. 20: 5'-ACGGCATCGTCAGTTGCG-3';
and
- optionally at least one oligonucleotide primer selected from the group consisting of:
SEQ ID No. 3: 5'-CGGAGATGAAAGCACCACTGTG-3'
SEQ ID No. 4: 5'-GGGCTGTGTAATCTCAGAGGAG-3'
SEQ ID No. 5: 5'-GTCCGGAGATGAAAGCACCACTGTG-3'
SEQ ID No. 6: 5'-TCAGGGCTGTGTAATCTCAGAGGAG-3'
SEQ ID No. 7: 5'-GGCGCTGATACCGGCAAGAATGG-3'
SEQ ID No. 8: 5'-GGTCCCGCAGGCCATGATT1TTG-3'
SEQ ID No. 9: 5'-CCGGCAAGAATGGTCGCAAACTCC-3'
SEQ ID No. 10: 5'-AAGGGGTTCCAAGCCGCAACTGACGA-3'
SEQ ID No. 11: 5'-TAAGGGGTTCCAAGCCGCAACTGACG-3'
SEQ ID No. 12: 5'-CTCAACGGCATCGTCAGTTGCGGCTTGGAAC-3'
SEQ ID No. 13: 5'-AGCACTCAACGGCATCGTCAGTTGCGGCTTG-3'
SEQ ID No. 14: 5'-CTATTTCAGGATACCCTTCGTCATCAACACG-3'
SEQ ID No. 15: 5'-AATTTCCCTTAATCCGGAGCTATTCGTATGA-3'SEQID
SEQ ID No. 16: 5'-GAAGACCAGCTTTTTGTTTC-3'
SEQ ID No. 17: 5'-TGTCACAGACTCAATGACTA-3'
SEQ ID No. 18: 5'-GGCATCGTCAGTTG-3'
SEQ ID No. 19: 5'-CGGCATCGTCAGTTGC-3'
SEQ ID No. 20: 5'-ACGGCATCGTCAGTTGCG-3'; and
for the detection of the amplified product.

35. Kit for the detection of *E. coli* 0157:H7 according to claim 34, comprising:
- two oligonucleotides of sequences SEQ ID No. 5 and SEQ ID No. 6, used as a pair of primers; and
- two oligonucleotides of sequences SEQ ID No. 15 and SEQ ID No. 18 for detection of the amplified product.

## Patentansprüche

1. Isolierte Nukleinsäure, die aus der Nukleotidsequenz SEQ ID NO: 1 oder der Nukleotidsequenz SEQ ID NO: 2, ihrer Komplementärsequenzen und den Fragmenten mit mindestens 8 aufeinanderfolgenden Nukleotiden der SEQ ID NO: 2 und der von SEQ ID NO: 2 abgeleitet Sequenzen, die sich durch Mutation, Insertion, Auslassung und/oder Substitution einer oder mehrerer Basen und Hybridisieren unter Bedingungen hoher Stringenz mit der Sequenz SEQ ID NO: 2 unterscheiden, besteht.

2. Isolierte Nukleinsäure bestehend aus einem Fragment mit mindestens 14 aufeinanderfolgenden Nukleotiden der Nukleotidsequenz SEQ ID NO: 1 oder einer Sequenz, die von der Nukleotidsequenz SEQ ID NO: 1 abgeleitet ist, die sich Auslassung und/oder Substitution einer oder mehrerer Basen und Hybridisieren unter Bedingungen hoher Stringenz mit der Sequenz SEQ ID NO: 1 unterscheidet, wobei das Fragment oder die abgeleitete Sequenz eine Nukleotidkette umfasst, die aus der stabilen Kombination von mindestens einem Abschnitt der Insertionssequenz IS91 und mindestens einem Abschnitt der Sequenz des katP Gens resultiert.

3. Isoliert Nukleinsäure gemäß Anspruch 1 oder 2, die mindestens 14 aufeinanderfolgende Nukleotide der Kette des Sequenz SEQ ID NO: 1, einschließlich der Nukleotide von Position 400 bis 407, umfasst.

4. Isolierte Nukleinsäure, die mindestens 8 aufeinanderfolgende Nukleotide des Sequenz SEQ ID NO: 2 oder der zu SEQ ID NO: 2 komplementären und davon abgeleiteten Sequenzen, wie in Anspruch 1 definiert, umfasst.

5. Isolierte Nukleinsäure gemäß Anspruch 4, die mindestens 14 aufeinanderfolgende Nukleotide der Sequenz SEQ ID NO: 2 umfasst.

6. Isolierte Nukleinsäure gemäß einem der Ansprüche 3 bis 5, die aus den folgenden Nukleotidsequenzen ausgewählt wird:
SEQ ID NO: 10: 5'-AAGGCGGTTCCAAGCCOCAACTGACGA-3'
SEQ ID NO: 11: 5'-TAAGGTGTTCCAAGCCGCAACTGACG-3'
SEQ ID NO: 12: 5'-CTCAACGGCATCGTCAGTTGCGGCTTGGAAC-3'
SEQ ID NO: 13: 5'-AGCACTCAACGGCATCGTCAGTTGCGGCTTG-3'
SEQ ID NO: 18: 5'-GGCATCGTCAGTTG-3'
SEQ ID NO: 19: 5'-CGGCATCGTCAGTTGC-3'
SEQ ID NO: 20: 5'-ACGGCATCGTCAGTTGCG-3
SEQ ID NO: 21: 5'-CCACCTGAACGATAAGCGGAAC-3'
SEQ ID NO: 22: 5'-CACCTTCCTTCCATCCTCAGAC-3'
SEQ ID NO: 23: 5'-ATCCCAGCGCGCTCCAGCTG-3'
SEQ ID NO: 24: 5'-ACCCATGATGGCGCATCTGATG-3'
SEQ ID NO: 25: 5'-ACGTTCTGGTCTTACGGGTGATGTAGGTTTT-3'
SEQ ID NO: 26: 5'-TAGTGAAGCGGTGACAGCATATCAGACGGCT-3'
SEQ ID NO: 27: 5'-GTGAGATAGGCACAACAATGA-3'.

7. Paare isolierter Nukleinsäuren, die als Primer verwendet werden, die aus den Paaren der folgenden Sequenzen ausgewählt werden:
SEQ ID NO: 3 und SEQ ID NO: 4
SEQ ID NO: 5 und SEQ ID NO: 6
SEQ ID NO: 6 und SEQ ID NO: 7
SEQ ID NO: 6 und SEQ ID NO: 8
SEQ ID NO: 6 und SEQ ID NO: 9
SEQ ID NO: 21 und SEQ ID NO: 22
SEQ ID NO: 23 und SEQ ID NO: 24.

8. Plasmide pDF3 und pDF4, die am 26. März 1998 am Collection Nationale de Cultures de Microorganismes unter den entsprechenden Nummern 1-1999 und 1-2000 hinterlegt wurde.

9. Wirtszelle, die ein Plasmid gemäß Anspruch 8 umfasst.

10. Ein Verfahren für das Nachweisen von enterohämorrhagischem *E. Coli* (EHEC) in einer Probe, wobei das Verfahren das Nachweisen einer Nukleotidkette der Sequenz SEQ ID NO: 2 mit einer isolierten Nukleinsäure, wie in Anspruch 4 definiert, umfasst, wobei das Vorhandensein der Nukleotidkette für das Vorhandensein eines EHEC bezeichnend ist.

11. Verfahren gemäß Anspruch 10, wobei die Sequenz der isoliert Nukleinsäure aus der Gruppe, die aus:
SEQ ID NO: 21: 5'-CCACCTGAACGATAAGCGGAAC-3'
SEQ ID NO: 22: 5'-CACCTTCCTTCCATCCTCAGAC-3'
SEQ ID NO: 23: 5'-ATCCCAGCGCGCTCCAGCTG-3'
SEQ ID NO: 24: 5'-ACCCATGATGGCGCATCTGATG-3'
SEQ ID NO: 25: 5'-ACGTTCTGGTCTTACGGGTGATGTAGGTTTT-3'
SEQ ID NO: 26: 5'-TAGTGAAGCGGTGACAGCATATCAGACGGCT-3' und
SEQ ID NO: 27: 5'-GTGAGATAGGCACAACAATGA-3"
besteht, ausgewählt wird.

12. Verfahren gemäß Anspruch 10 oder 11, wobei die isolierte Nukleinsäure als ein Primer und/oder eine Sonde verwendet wird.

13. Verfahren gemäß einem der Ansprüche 10 bis 12, wobei die isolierte Nukleinsäure markiert wird.

14. Verfahren gemäß einem der Ansprüche 10 bis 13, wobei die isolierte Nukleinsäure auf einem Träger immobilisiert ist.

15. Verfahren zum Nachweisen von EHEC in einer Probe, das die folgenden Schritte umfasst:
(a) In-Kontakt-bringen der Probe mit einem Paar von Oligonukleotidprimern, die aus der Gruppe, die aus
SEQ ID NO: 21: 5'-CCACCTGAACGATAAGCGGAAC-3'
SEQ ID NO: 22: 5'-CACCTTCCTTCCATCCTCAGAC-3'
SEQ ID NO: 23: 5'-ATCCCAGCGCGCTCCAGCTG-3'
SEQ ID NO: 24: 5'-ACCCATGATGGCGCATCTGATG-3'
SEQ ID NO: 25: 5'-ACGTTCTGGTCTTACGGGTGATGTAGGTTTT-3'
SEQ ID NO: 26: 5'-TAGTGAAGCGGTGACAGCATATCAGACGGCT-3' und
SEQ ID NO: 27: 5'-GTGAGATAGGCACAACAATGA-3'
besteht ausgewählt wird; wobei die Nukleinsäure, die in der Probe enthalten ist, den Primern, wo geeignet, an dem untersuchten Target zugänglich gemacht wurde,
(b) Amplifizieren der Nukleotidsequenz, die von dem Paar der gewählten Primern flankiert ist,
(c) Verifizierung des Vorhandenseins des amplifizierten Produkts unter Verwendung von mindestens einer Sonde, die für das amplifizierte Produkt spezifisch ist.

16. Verfahren gemäß Anspruch 15, gemäß dem Schritt (c) die folgenden Teilschritte umfasst:
(c₁) Denaturieren der amplifizierten Sequenzen durch ein physikalisches oder chemisches Mittel,
(c₂) In-Kontakt-bringen einer Lösung, die die denaturierten amplifizierten Fragmente von Schritt (c₁) enthält, mit einerseits mindestens einer Fangsonde und andererseits mindestens einer Nachweissonde, die optional markiert ist, wobei die Fang- und Nachweissonden mit dem gleichen Strang der amplifizierten Fragmente hybridisieren können, wobei das In-Kontakt-bringen über einen Zeitraum durchgeführt wird, der ausreichend ist, um die Hybridisierung-Reaktion zu ermöglichen,
(c₃) mindestens einmaliges Waschen, um die nicht umgesetzten Nukleotidsequenzen zu entfernen,
(c₄) Visualisieren der Nachweissonden, die mit den amplifizierten Nukleotidsequenzen hybridisiert sind.

17. Verfahren gemäß Anspruch 16, bei dem die Fangsonde an der Oberfläche einer Vertiefung einer Mikrotiterplatte angebracht ist.

18. Das Verfahren gemäß Anspruch 16 oder 17, bei dem die Nachweissonde mit Peroxidase markiert ist.

19. Verfahren gemäß Anspruch 18, **dadurch gekennzeichnet, dass** das Nachweisen der Aktivität der Peroxidase, die mit der Nachweissonde, die reagiert hat, verbunden ist, durch kolorimetrische Reaktion in Gegenwart eines chromogenen Substrats, wie Tetramethylbenzidin (TMB), unter Anwendung der folgenden Schritten durchgeführt wird:
- Hinzufügen des chromogenen Substrats, wie eine TMB Lösung, in die Vertiefungen, die das Reaktionsgemisch enthalten,
- Inkubieren in der Dunkelheit über einen ausreichenden Zeitraum, damit sich die Farbe entwickeln kann,
- Blockieren der Reaktion durch Hinzufügen einer Blockier-Lösung,
- Bestimmen der optischen Dichte bei einer geeigneten Wellenlänge.

20. Verfahren zum Nachweisen des EHEC, gemäß einem der Ansprüche 16 bis 19 unter Verwendung der folgenden Oligonukleotide:
- die Sequenzen SEQ ID NO: 21 und SEQ ID NO: 22 als Primer für die Amplifikation,
- die Sequenz SEQ ID NO: 25 als Fangsonde, und
- die Sequenz SEQ ID NO: 27 als Nachweissonde.

21. Verfahren zum Nachweisen von *E. Coli* O157:H7 in einer Probe, wobei das Verfahren das Nachweisen einer Nukleotidkette umfasst, die aus der stabilen Kombination von mindestens einem Abschnitt der Insertions-Sequenz IS91 und von mindestens einem Abschnitt der Sequenz des *katP* Gens unter Zuhilfenahme einer isoliert Nukleinsäure resultiert, die mindestens 8 aufeinanderfolgende Nukleotide der Sequenz SEQ ID NO: 1, der dazu komplementären Sequenz oder eine davon abgeleitete Sequenz, die sich durch Auslassung und/oder Substitution von einer oder mehreren Basen und Hybridisieren unter Bedingungen hoher Stringenz mit der Sequenz SEQ ID NO: 1 unterscheidet, umfasst, wobei das Vorhandensein der Nukleotidkette für das Vorhandensein von *E. Coli* O157:H7 bezeichnend ist.

22. Das Verfahren gemäß Anspruch 21, wobei die Sequenz der isoliert Nukleinsäure aus der Gruppe, die aus:
SEQ ID NO: 3: 5'-CGGAGATGAAAGCACCACTGTG-3'
SEQ ID NO: 4: 5'-GGGCTGTGTAATCTCAGAGGAG-3'
SEQ ID NO: 5: 5'-GTCCGGAGATGAAAGCACCACTGTG-3'
SEQ ID NO: 6: 5'-TCAGGGCTGTGTAATCTCAGAGGAG-3'
SEQ ID NO: 7: 5'-GGCGCTGATACCGGCAAGAATGG-3'
SEQ ID NO: 8: 5'-GGTCCCGCAGGCCATGATTTTTG-3'
SEQ ID NO: 9: 5'-CCGGCAAGAATGGTCGCAAACTCC-3'
SEQ ID NO: 10: 5'-AAGGGGTTCCAAGCCGCAACTGACGA-3'
SEQ ID NO: 11: 5'-TAAGGGGTTCCAAGCCGCAACTGACG-3'
SEQ ID NO: 12: 5'-CTCAACGGCATCGTCAGTTGCGGCTTGGAAC-3'
SEQ ID NO: 13: 5'-AGCACTCAACGGCATCGTCAGTTGCGGCTTG-3'
SEQ ID NO: 14: 5'-CTATTTCAGGATACCCTTCGTCATCAACACG-3'
SEQ ID NO: 15: 5'-AATTTCCCTTAATCCGGAGCTATTCGTATGA-3'
SEQ ID NO: 16: 5'-GAAGACCAGCTTTTTGTTTC-3'
SEQ ID NO: 17: 5'-TGTCACAGACTCAATGACTA-3'
SEQ ID NO: 18: 5'-GGCATCGTCAGTTG-3'
SEQ ID NO: 19: 5'-CGGCATCGTCAGTTGC-3' und
SEQ ID NO: 20: 5'-ACGGCATCGTCAGTTGCG-3'
besteht, ausgewählt wird.

23. Das Verfahren gemäß einem der Ansprüche 21 oder 22, wobei die isolierte Nukleinsäure als Primer und/oder Sonde verwendet wird.

24. Das Verfahren gemäß einem der Ansprüche 21 bis 23, wobei die isolierte Nukleinsäure markiert ist.

25. Das Verfahren gemäß einem der Ansprüche 21 bis 23, wobei die isolierte Nukleinsäure auf einem Träger immobilisiert ist.

26. Das Verfahren gemäß Anspruch 21, das die folgenden Schritte umfasst:
(a) In-Kontakt-bringen der Probe mit einem Paar von Oligonukleotidprimern, die aus der Gruppe, die aus:
SEQ ID NO: 3: 5'-CGGAGATGAAAGCACCACTGTG-3'
SEQ ID NO: 4: 5'-GGGCTGTGTAATCTCAGAGGAG-3'
SEQ ID NO: 5: 5'-GTCCGGAGATGAAAGCACCACTGTG-3'
SEQ ID NO: 6: 5'-TCAGGGCTGTGTAATCTCAGAGGAG-3'
SEQ ID NO: 7: 5'-GGCGCTGATACCGGCAAGAATGG-3'
SEQ ID NO: 8: 5'-GGTCCCGCAGGCCATGATTTTTG-3'
SEQ ID NO: 9: 5'-CCGGCAAGAATGGTCGCAAACTCC-3'
SEQ ID NO: 10: 5'-AAGGGGTTCCAAGCCGCAACTGACGA-3'
SEQ ID NO: 11: 5'-TAAGGGGTTCCAAGCCGCAACTGACG-3'
SEQ ID NO: 12: 5'-CTCAACGGCATCGTCAGTTGCGGCTTGGAAC-3'
SEQ ID NO: 13: 5'-AGCACTCAACGGCATCGTCAGTTGCGGCTTG-3'
SEQ ID NO: 18: 5'-GGCATCGTCAGTTG-3'
SEQ ID NO: 19: 5'-CGGCATCGTCAGTTGC-3' und
SEQ ID NO: 20: 5'-ACGGCATCGTCAGTTGCG-3'
besteht, ausgewählt wird, wobei die Nukleinsäure, die in der Probe enthalten ist, den Primern, wo geeignet, an dem untersuchten Target zugänglich gemacht wurde,
(b) Amplifizieren der Nukleotidsequenz, die von dem Paar der gewählten Primer flankiert sind,
(c) Verifizierung des Vorhandenseins des amplifizierten Produkts unter Verwendung von mindestens einer Sonde, die für das amplifizierte Produkt spezifisch ist.

27. Das Verfahren gemäß Anspruch 26, gemäß dem Schritt (c) die folgenden Teilschritte umfasst:
(c₁) Denaturieren der amplifizierten Sequenzen durch ein physikalisches oder chemisches Mittel,
(c₂) In-Kontakt-bringen einer Lösung, die die denaturierten amplifizierten Fragmente von Schritt (c₁) enthält, mit einerseits mindestens einer Fangsonde und andererseits mindestens einer Nachweissonde, die optional markiert ist, wobei die Fang- und Nachweissonden mit dem gleichen Strang der amplifizierten Fragmente hybridisieren können, wobei das In-Kontakt-bringen über einen Zeitraum durchgeführt wird, der ausreichend ist, um die Hybridisierung-Reaktion zu ermöglichen,
(c₃) mindestens einmaliges Waschen, um die nicht umgesetzten Nukleotidsequenzen zu entfernen,
(c₄) Visualisieren der Nachweissonden, die mit den amplifizierten Nukleotidsequenzen hybridisiert sind.

28. Das Verfahren gemäß Anspruch 27, bei dem die Fangsonde an der Oberfläche einer Vertiefung einer Mikrotiterplatte angebracht ist.

29. Das Verfahren gemäß Anspruch 27 oder 28, bei dem die Nachweissonde mit Peroxidase markiert ist.

30. Das Verfahren gemäß Anspruch 29, **dadurch gekennzeichnet, dass** das Nachweisen der Aktivität der Peroxidase, die mit der Nachweissonde, die reagiert hat, verbunden ist, durch kolorimetrische Reaktion in Gegenwart eines chromogenen Substrats, wie Tetramethylbenzidin (TMB), unter Anwendung der folgenden Schritten durchgeführt wird:
- Hinzufügen des chromogenen Substrats, wie eine TMB Lösung, in die Vertiefungen, die das Reaktionsgemisch enthalten,
- Inkubieren in der Dunkelheit über einen ausreichenden Zeitraum, damit sich die Farbe entwickeln kann,
- Blockieren der Reaktion durch Hinzufügen einer Blockier-Lösung,
- Bestimmen der optischen Dichte bei einer geeigneten Wellenlänge.

31. Verfahren zum Nachweisen von *E. Coli* O157:H7 gemäß einem der Ansprüchen 27 bis 30 unter Verwendung der folgenden Oligonukleotide:
- die Sequenzen SEQ ID NO: 5 und SEQ ID NO: 6 als Primer für die Amplifikation,
- die Sequenz SEQ ID NO: 15 als Fangsonde,
- die Sequenz SEQ ID NO: 18 als Nachweissonde.

32. Kit für das Nachweisen von EHECs, das unter den Reagenzien umfasst:
- mindestens zwei Nukleotid-Primer, die aus der Gruppe, die aus:
SEQ ID NO: 21: 5'-CCACCTGAACGATAAGCGGAAC-3'
SEQ ID NO: 22: 5'-CACCTTCCTTCCATCCTCAGAC-3'
SEQ ID NO: 23: 5'-ATCCCAGCGCGCTCCAGCTG-3'
SEQ ID NO: 24: 5'-ACCCATGATGGCGCATCTGATG-3'
SEQ ID NO: 25: 5'-ACGTTCTGGTCTTACGGGTGATGTAGGTTTT-3'
SEQ ID NO: 26: 5'-TAGTGAAGCGGTGACAGCATATCAGACGGCT-3' und
SEQ ID NO: 27: 5'-GTGAGATAGGCACAACAATGA-3'
besteht, ausgewählt wird, und
- optional mindestens eine Nukleotid-Sonde, die aus der Gruppe, die aus:
SEQ ID NO: 21: 5'-CCACCTGAACGATAAGCGGAAC-3'
SEQ ID NO: 22: 5'-CACCTTCCTTCCATCCTCAGAC-3'
SEQ ID NO: 23: 5'-ATCCCAGCGCGCTCCAGCTG-3'
SEQ ID NO: 24: s, ACCCATGATGGCGCATCTGATG-3'
SEQ ID NO: 25: 5'-ACGTTCTGGTCTTACGGGTGATGTAGGTTTT-3'
SEQ ID NO: 26: 5'-TAGTGAAGCGGTGACAGCATATCAGACGGCT-3' und
SEQ ID NO: 27: 5'-GTGAGATAGGCACAACAATGA-3'
besteht, ausgewählt wird, für das Nachweisen des amplifizierten Produkts.

33. Kit für das Nachweisen von EHECs gemäß Anspruch 32, das umfasst:
- zwei Oligonukleotide der Sequenz SEQ ID NO: 21 und SEQ ID NO: 22, die als ein Paar von Primern verwendet werden,
- zwei Oligonukleotide der Sequenz SEQ ID NO: 25 und SEQ ID NO: 27 für das Nachweisen des amplifizierten Produkts.

34. Kit für das Nachweisen von *E. Coli* O157;H7, das unter den Reagenzien umfasst:
- mindestens zwei Oligonucleotide-Primer, die aus der Gruppe, die aus:
SEQ ID NO: 3: 5'-CGGAGATGAAAGCACCACTGTG-3'
SEQ ID NO: 4: 5'-GGGCTGTGTAATCTCAGAGGAG-3'
SEQ ID NO: 5: 5'-GTCCGGAGATGAAAGCACCACTGTG-3'
SEQ ID NO: 6: 5'-TCAGGGCTGTGTAATCTCAGAGGAG-3'
SEQ ID NO: 7: 5'-GGCGCTGATACCGGCAAGAATGG-3'
SEQ ID NO: 8: 5'-GGTCCCGCAGGCCATGATTTTTG-3'
SEQ ID NO: 9: 5'-CCGGCAAGAATGGTCGCAAACTCC-3'
SEQ ID NO: 10: 5'-AAGGGGTTCCAAGCCGCAACTGACGA-3'
SEQ ID NO: 11: 5'-TAAGGGGTTCCAAGCCGCAACTGACG-3'
SEQ ID NO: 12: 5'-CTCAACGGCATCGTCAGTTGCGGCTTGGAAC-3'
SEQ ID NO: 13: 5'-AGCACTCAACGGCATCGTCAGTTGCGGCTTG-3'
SEQ ID NO: 14: 5'-CTATTTCAGGATACCCTTCGTCATCAACACG-3'
SEQ ID NO: 15: s, AATTTCCCTTAATCCGGAGCTATTCGTATGA-3'
SEQ ID NO: 16: 5'-GAAGACCAGCTTTTTGTTTC-3'
SEQ ID NO: 17: 5'-TGTCACAGACTCAATGACTA-3'
SEQ ID NO: 18: 5'-GGCATCGTCAGTTG-3'
SEQ ID NO: 19: 5'-CGGCATCGTCAGTTGC-3' und
SEQ ID NO: 20: 5'-ACGGCATCGTCAGTTGCG-3'
besteht, ausgewählt wird, und
- optional mindestens eine Oligonukleotid-Sonde, die aus der Gruppe, die aus:
SEQ ID NO: 3: 5'-CGGAGATGAAAGCACCACTGTG-3'
SEQ ID NO: 4: 5'-GGGCTGTGTAATCTCAGAGGAG-3'
SEQ ID NO: 5: 5'-GTCCGGAGATGAAAGCACCACTGTG-3'
SEQ ID NO: 6: 5'-TCAGGGCTGTGTAATCTCAGAGGAG-3'
SEQ ID NO: 7: 5'-GGCGCTGATACCGGCAAGAATGG-3'
SEQ ID NO: 8: 5'-GGTCCCGCAGGCCATGATTTTTG-3'
SEQ ID NO: 9: 5'-CCGGCAAGAATGGTCGCAAACTCC-3'
SEQ ID NO: 10: 5'-AAGGGGTTCCAAGCCGCAACTGACGA-3'
SEQ ID NO: 11: 5'-TAAGGGGTTCCAAGCCGCAACTGACG-3'
SEQ ID NO: 12: 5'-CTCAACGGCATCGTCAGTTGCGGCTTGGAAC-3'
SEQ ID NO: 13: 5'-AGCACTCAACGGCATCGTCAGTTGCGGCTTG-3'
SEQ ID NO: 14: 5'-CTATTTCAGGATACCCTTCGTCATCAACACG-3'
SEQ ID NO: 15: 5'-AATTTCCCTTAATCCGGAGCTATTCGTATGA-3'
SEQ ID NO: 16: 5'-GAAGACCAGCTTTTTGTTTC-3'
SEQ ID NO: 17: 5'-TGTCACAGACTCAATGACTA-3'
SEQ ID NO: 18: 5'-GGCATCGTCAGTTG-3'
SEQ ID NO: 19: 5'-CGGCATCGTCAGTTGC-3' und
SEQ ID NO: 20: 5'-ACGGCATCGTCAGTTGCG-3'
besteht, ausgewählt wird, für das Nachweisen des amplifizierten Produkts.

35. Kit für das Nachweisen von *E. Coli* O157:H7 gemäß Anspruch 34, das umfasst :
- zwei Oligonukleotide der Sequenz SEQ ID NO: 5 und SEQ ID NO: 6, die als Paar von Primern verwendet werden,
- zwei Oligonucleotide der Sequenz SEQ ID NO: 15 und SEQ ID NO: 18, für das Nachweisen des amplifizierten Produkts.
